# FASCICULE DE BREVET EUROPEEN

(11) **EP 2 364 311 B1**
(45) Date de publication et mention de la délivrance du brevet: **03.07.2013**
(21) Numéro de dépôt: 09760218.9
(22) Date de dépôt: 27.10.2009
(51) Int. Cl.: C07D 451/02, C07D 451/06, C07D 471/08, A61K 31/496, A61P 3/00, A61P 9/00, A61P 19/00, A61P 25/00

(54) **DERIVES D'UREES DE TROPANE, LEUR PREPARATION ET LEUR APPLICATION EN THERAPEUTIQUE COMME MODULATEURS DE L'ACTIVITE DE LA 11BETAHSD1**
TROPANHARNSTOFFDERIVATE, DEREN HERSTELLUNG UND DEREN THERAPEUTISCHE VERWENDUNG ALS MODULATOREN DER 11BETAHSD1-AKTIVITÄT
TROPANE UREA DERIVATIVES, PREPARATION THEREOF AND THERAPEUTIC APPLICATION THEREOF AS MODULATORS OF THE ACTIVITY OF 11BETAHSD1

(30) Priorité: 28.10.2008 FR 0805974
(43) Date de publication de la demande: 14.09.2011
(73) Titulaire: SANOFI, 75008 Paris (FR)
(72) Inventeur: BRAUN, Alain, Jean, F-75013 Paris (FR); CRESPIN, Olivier, F-75013 Paris (FR); NICOLAI, Eric, F-75013 Paris (FR); PASCAL, Cécile, F-75013 Paris (FR); VENIER, Olivier, F-75013 Paris (FR)
(74) Mandataire: Böhm, Brigitte
(86) Numéro de dépôt international: PCT/FR2009/052060
(87) Numéro de publication internationale: WO 2010/049635

(56) Documents cités:
- WO-A-2005/108368
- WO-A-2007/063071
- WO-A1-2005/028477
- WO-A1-2006/045716
- WO-A1-2009/067579
- FR-A- 2 902 791
- GB-A- 1 164 555
- US-A1- 2005 065 178
- KRUNIC ET AL: "Synthesis and monoamine transporter affinity of 3-aryl substituted trop-2-enes" BIOORGANIC & MEDICINAL CHEMISTRY LETTERS, PERGAMON, ELSEVIER SCIENCE, GB, vol. 15, no. 24, 15 décembre 2005 (2005-12-15), pages 5488-5493, XP005136469 ISSN: 0960-894X
- DATABASE CA [Online] CHEMICAL ABSTRACTS SERVICE, COLUMBUS, OHIO, US; 1970, NADOR, KAROLY ET AL: "Syntheses and the stereochemistry of tropanyl ethers" XP002573914 extrait de STN Database accession no. 1970:90243 & KEMIAI KOZLEMENYEK , 31(4), 351-81 CODEN: KEKOAS; ISSN: 0022-9814, 1969,

## Description

La présente invention se rapporte à des dérivés d'urée de tropane, à leur préparation et à leur application en thérapeutique. Les présents composés modulent l'activité de la 11β-hydroxystéroïde déhydrogénase type 1 (11βHSD1) et sont utiles pour le traitement des pathologies dans lesquelles une telle modulation est bénéfique, comme dans le cas du syndrome métabolique ou du diabète de type 2 non insulino dépendant.

La 11β-hydroxystéroïde déhydrogénase type 1 (11βHSD1) catalyse localement la conversion de glucocorticoïdes inactifs (cortisone chez l'homme) en glucocorticoïdes actifs (cortisol chez l'homme) dans différents tissus et organes, principalement le foie et le tissu adipeux, mais aussi dans les muscles, les os, le pancréas, l'endothélium, le tissu oculaire et dans certaines parties du système nerveux central. La 11βHSD1 agit comme un régulateur de l'action des glucocorticoïdes dans les tissus et organes où elle est exprimée (Tomlinson et al., Endocrine Reviews 25(5), 831-866 (2004), Davani et al., J. Biol. Chem. 275, 34841 (2000) ; Moisan et al., Endocrinology, 127, 1450 (1990) ).

Les pathologies les plus importantes dans lesquelles interviennent les glucocorticoïdes et l'inhibition de la 11βHSD1 sont indiquées ci-après.

### A. Obésité, diabète de type 2 et syndrome métabolique

Le rôle de la 11βHSD1 dans l'obésité, le diabète de type 2 et le syndrome métabolique (aussi connu sous le nom de syndrome X ou syndrome de résistance à l'insuline) où les symptômes incluent l'obésité viscérale, l'intolérance au glucose, la résistance à l'insuline, l'hypertension, le diabète de type 2 et l'hyperlipidémie (Reaven Ann. Rev. Med 44, 121 (1993)) est décrit dans de nombreuses publications. Chez l'homme, le traitement par la carbenoxolone (un inhibiteur non spécifique de la 11βHSD1) améliore la sensibilité à l'insuline chez des patients volontaires minces et chez des diabétiques de type 2 (Andrews et al., J. Clin. Endocrinol. Metab. 88, 285 (2003)). De plus les souris, dont le gène de la 11βHSD1 a été éteint, sont résistantes à l'hyperglycémie induite par le stress et l'obésité, montrent une atténuation de l'induction d'enzymes hépatiques de la néoglucogenèse (PEPCK et G6P) et présentent une augmentation de la sensibilité à l'insuline dans le tissu adipeux (Kotelevstev et al., Proc. Nat Acad. Sci. 94, 14924 (1997) ; Morton et al., J. Biol. Chem. 276, 41293 (2001)). Par ailleurs, les souris transgéniques où le gène de la 11βHSD1 a été surexprimé dans les tissus adipeux présentent un phénotype similaire à celui du syndrome métabolique humain (Masuzaki et al., Science 294, 2166 (2001)). Il est à noter que le phénotype observé existe sans une augmentation du total des glucocorticoides circulants, mais est induit par l'augmentation spécifique de glucocorticoides actifs dans les dépôts adipeux.

Par ailleurs, de nouvelles classes d'inhibiteurs spécifiques de la 11βHSD1, sont apparues récemment :
- des arylsulfonamidothiazoles ont montré qu'ils amélioraient la sensibilité à l'insuline et réduisaient le niveau de glucose dans le sang de souris présentant une hyperglycémie (Barf et al., J. Med. Chem. 45, 3813 (2002)). De plus, dans une étude récente, il a été montré que ce type de composés réduisait la prise de nourriture ainsi que la prise de poids chez des souris obèses (Wang et Coll. Diabetologia 49, 1333 (2006*)).*
- des triazoles ont montré qu'ils amélioraient le syndrome métabolique et ralentissaient la progression de l'athérosclérose chez des souris (Hermanowski-Vosatka et al., J. Exp. Med. 202, 517 (2005)).

### B. Cognition et démence

Les problèmes cognitifs légers sont des phénomènes communs chez les personnes âgées et peuvent conduire finalement à la progression de la démence. Autant dans le cas d'animaux que d'humains âgés, les différences inter-individuelles pour les fonctions cognitives générales ont été reliées aux différences d'exposition à long terme aux glucocorticoïdes (Lupien et al., Nat. Neurosci. 1, 69, (1998)). Par ailleurs, la dérégulation de l'axe HPA (hypothalamo-hypophysio-surrénalien)) résultant dans l'exposition chronique aux glucocorticoïdes de certaines sous-régions du cerveau a été proposée comme contribuant au déclin des fonctions cognitives (Mc Ewen et al., Curr. Opin. Neurobiol. 5, 205, 1995). La 11βHSD1 est abondante dans le cerveau et est exprimée dans de nombreuses sous régions incluant l'hypothalamus, le cortex frontal et le cerebellum (Sandeep et al., Proc. Natl. Acad. Sci. 101, 6734 (2004)). Les souris déficientes en 11βHSD1 sont protégées contre les dysfonctionnements de l'hypothalamus associés aux glucocorticoïdes qui sont rattachés à la vieillesse (Yau et al., Proc. Natl. Acad. Sci. 98, 4716, (2001)). De plus, dans des études chez l'homme, il a été montré que l'administration de la carbenoxolone améliore la fluidité verbale et la mémoire verbale chez les personnes âgées (Yau et al., Proc. Natl. Acad. Sci. 98, 4716 (2001), Sandeep et al., Proc. Natl. Acad. Sci. 101, 6734 (2004)). Finalement, l'utilisation d'inhibiteurs sélectifs de la 11βHSD1 de type triazole a montré qu'ils prolongeaient la rétention de la mémoire chez des souris âgées (Rocha et al., Abstract 231 ACS meeting, Atlanta, 26-30 Mars 2006).

### C. Pression intra-oculaire

Les glucocorticoïdes peuvent être utilisés par voies topique ou systémique pour une grande variété de pathologies de l'ophtalmologie clinique. Une complication particulière de ces traitements est le glaucome induit par l'utilisation de corticostéroïdes. Cette pathologie est caractérisée par l'augmentation de la pression intra-oculaire (PIO). Dans les cas les plus graves et pour les formes non traitées, la PIO peut conduire à une perte de champ de vision partielle et éventuellement à une perte totale de la vision. La PIO est le résultat d'un déséquilibre entre la production d'humeur aqueuse et son drainage. L'humeur aqueuse est produite dans les cellules épithéliales non-pigmentées et le drainage est réalisé au travers des cellules du réseau trabéculaire. La 11βHSD1 est localisée dans les cellules épithéliales non-pigmentées et sa fonction est clairement l'amplification de l'activité des glucocorticoïdes dans ces cellules (Stokes et al., Invest. Ophthalmol. Vis. Sci. 41, 1629 (2000)). Cette notion est confirmée par l'observation que la concentration en cortisol libre est fortement excédentaire par rapport à la cortisone dans l'humeur aqueuse (ratio 14/1) . L'activité fonctionnelle de la 11βHSD1 dans les yeux a été évaluée en étudiant l'action de la carbenoxolone chez des volontaires sains. Après sept jours de traitement à la carbenoxolone, la PIO est réduite de 18% (Rauz et al., Invest. Ophtamol. Vis. Sci. 42, 2037 (2001)). L'inhibition de la 11βHSD1 dans les yeux est donc prédite comme réduisant la concentration locale en glucocorticoïdes et la PIO, produisant un effet bénéfique dans le traitement du glaucome et d'autres désordres de la vision.

### D. Hypertension

Les substances hypertensives issues des adipocytes comme la leptine et l'angiotensinogène ont été proposées comme étant des éléments clés dans les pathologies d'hypertension reliées à l'obésité (Wajchenberg et al., Endocr. Rev. 21, 697 (2000)). La leptine qui est secrétée en excès chez les souris aP2-11βHSD1 transgéniques (Masuzaki et al., J. Clinical Invest. 112, 83 (2003)), peut activer différents réseaux de systèmes neuronaux sympathiques, incluant ceux qui régulent la pression artérielle (Matsuzawa et al., Acad. Sci. 892, 146 (1999)). De plus, le système rénine-angiotensine (SRA) a été identifié comme étant une voie déterminante dans la variation de la pression artérielle. L'angiotensinogène, qui est produit dans le foie et le tissu adipeux, est un substrat-clé pour la rénine et est à l'origine de l'activation du SRA. Le niveau plasmatique en angiotensiogène est significativement élevé dans les souris aP2-11βHSD1 transgéniques, comme le sont ceux de l'angiotensine II et de l'aldostérone (Masuzaki et al., J Clinical Invest. 112, 83 (2003)) ; ces éléments conduisent à l'élévation de la pression artérielle. Le traitement de ces souris par de faibles doses d'un antagoniste du récepteur de l'angiotensine II abolit cette hypertension (Masuzaki et al., J. Clinical Invest. 112, 83 (2003)). Ces informations illustrent l'importance de l'activation locale des glucocorticoïdes dans le tissu adipeux et le foie, et suggère que cette hypertension puisse être causée ou exacerbée par l'activité de la 11βHSD1 dans ces tissus. L'inhibition de la 11βHSD1 et la réduction du niveau de glucocorticoïdes dans le tissu adipeux et/ou dans le foie est donc prédit comme ayant un rôle bénéfique pour le traitement de l'hypertension et des pathologies cardiovasculaires associées.

### E. Ostéoporose

Le développement du squelette et les fonctions osseuses sont aussi régulées par l'action des glucocorticoïdes. La 11βHSD1 est présente dans les ostéoclastes et ostéoblastes. Le traitement de volontaires sains par la carbenoxolone a montré une diminution des marqueurs de résorption osseuse sans changement dans les marqueurs de formation des os (Cooper et al., Bone, 27, 375 (2000)). L'inhibition de la 11βHSD1 et la réduction du niveau de glucocorticoïdes dans les os pourraient donc être utilisées comme un mécanisme de protection dans le traitement de l'ostéoporose.

FR 2902791 décrit des dérivés d'urée de tropane comme inhibiteurs spécifiques de la 11βHSD1.

On a maintenant trouve des dérivés d'urées de tropane qui modulent l'activité de la 11betaHSD1.

La présente invention a pour objet des composés répondant à la formule (I) : 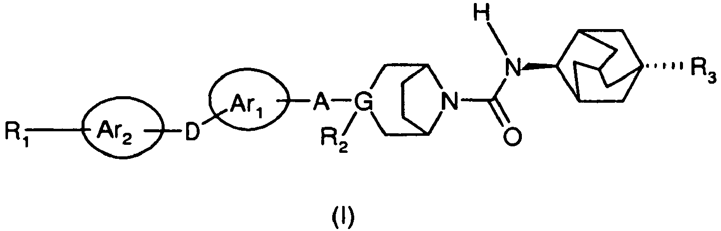 A représente une liaison ou un groupe -O-(CH₂)ₙ- avec n est un nombre égal 30 à 0 ou 1,
D représente une liaison ou un atome d'oxygène,
G représente un atome de carbone ou d'azote,
Ar1 représente un groupe aryle ou hétéroaryle,
Ar2 représente un groupe aryle ou hétéroaryle ou hétérocycloalkyle,
R1 représente un atome d'hydrogène, un groupe (C1-C6)alkyle, -SO₂-(C1-C6)alkyle, -SO₂-halogéno(C1-C6)alkyle, (C1-C6)alcoxy ou -Si(alkyle)₃,
R2 représente un atome d'hydrogène ou un groupe hydroxy ou, quand G est un atome d'azote, R2 est absent,
R3 représente un groupe hydroxy ou -C(O)-NH₂.

Parmi les composés décrits dans la présente invention, on peut citer un premier groupe de composés répondant à la formule (I) dans laquelle :
A représente une liaison ou un groupe -O-(CH₂)ₙ-avec n est un nombre égal à 0 ou 1,
   et/ou
G représente un atome de carbone ou d'azote,
   et/ou
D représente une liaison ou un atome d'oxygène,
   et/ou
Ar1 représente un groupe aryle ou hétéroaryle,
   et/ou
Ar2 représente un groupe aryle ou hétéroaryle ou hétérocycloalkyle,
   et/ou
R1 représente un atome d'hydrogène, un groupe (C1-C6)alkyle, -SO₂-(C1-C6)alkyle, -S0₂-halogéno(C1-C6)alkyle, (C1-C6)alcoxy ou -Si(alkyle)₃ ;
   et/ou
R2 représente un atome d'hydrogène ou un groupe hydroxy ou, quand G est un atome d'azote, R2 est absent,
   et/ou
R3 représente un groupe hydroxy ou -C(O)-NH₂.

Les composés de formule (I) peuvent comporter un ou plusieurs atomes de carbone asymétriques. Ils peuvent donc exister sous forme d'énantiomères ou de diastéréoisomères. Quand G représente un atome de carbone, les composés de formule (I) peuvent exister sous forme endo ou exo. Ces énantiomères, diastéréoisomères, forme endo ou exo, ainsi que leurs mélanges, y compris les mélanges racémiques, font partie de l'invention.

Les composés de formule (I) peuvent exister à l'état de bases ou salifiés par des acides ou des bases, notamment des acides ou des bases pharmaceutiquement acceptables. De tels sels d'addition font partie de l'invention.

Ces sels sont avantageusement préparés avec des acides pharmaceutiquement acceptables, mais les sels d'autres acides utiles, par exemple, pour la purification ou l'isolement des composés de formule (I), font également partie de l'invention.

Dans le cadre de la présente invention, et sauf mention différente dans le texte, on entend par :
- un atome d'halogène : un fluor, un chlore, un brome ou un iode ;
- un groupe alkyle : un groupe aliphatique saturé linéaire ou ramifié de 1 à 6 atomes de carbone. A titre d'exemples, on peut citer les groupes méthyle, éthyle, propyle, isopropyle, butyle, isobutyle, tertbutyle, pentyle, etc ;
- un groupe hétérocycloalkyle : un groupe alkyle cyclique comprenant entre 3 et 6 atomes de carbone et comprenant entre 1 et 3 hétéroatomes, tels que l'azote, l'oxygène ou le soufre. A titre d'exemples, on peut citer les groupes hétérocycloalkyles monocycliques tel que le groupe pipérazine, morpholine, etc ;
- un groupe alcoxy : un radical -O-alkyle où le groupe alkyle est tel que précédemment défini ;
- un groupe halogénoalkyle : un groupe alkyle dont un ou plusieurs atomes d'hydrogène ont été substitués par un atome d'halogène. A titre d'exemples, on peut citer le groupe -CH₂-CF₃;
- un groupe aryle : un groupe aromatique cyclique comprenant entre 5 et 6 atomes de carbone. A titre d'exemples de groupes aryles, on peut citer le groupe phényle ;
- un groupe hétéroaryle : un groupe aromatique cyclique comprenant entre 5 et 6 atomes de carbone et comprenant entre 1 et 3 hétéroatomes, tels que l'azote, l'oxygène ou le soufre. A titre d'exemples de groupes hétéroaryles, on peut citer le groupe pyridine, le groupe pyrimidine.

Un premier sous-groupe de composés de l'invention est constitué par les composés de formule (I) dans laquelle les substituants du groupe Ar1 sont situés en position para l'un par rapport à l'autre.

Un deuxième sous-groupe de composés de l'invention est constitué par les composés de formule (I) dans laquelle les substituants du groupe Ar2 sont situés en position para l'un par rapport à l'autre.

Un troisième sous-groupe de composés de l'invention est constitué par les composés de formule (I) dans laquelle Ar1 représente un groupe phényle, pyridine ou pyrimidine.

Un quatrième sous-groupe de composés de l'invention est constitué par les composés de formule (1) dans laquelle Ar2 représente un groupe phényle, pyridine, pipérazine ou morpholine.

Un cinquième sous-groupe de composés de l'invention est constitué par les composés de formule (I) dans laquelle G représente un atome de carbone.

Un sixième sous-groupe de composés de l'invention est constitué par les composés de formule (I) dans laquelle G représente un atome de carbone et A est un atome d'oxygène.

Un septième sous-groupe de composés de l'invention est constitué par les composés de formule (I) dans laquelle G représente un atome de carbone et A représente un groupe -O-(CH₂)ₙ-avec n égal à 1.

Un huitième sous-groupe de composés de l'invention est constitué par les composés de formule (I) dans laquelle G représente un atome de carbone et A est une liaison.

Un neuvième sous-groupe de composés de l'invention est constitué par les composés de formule (I) dans laquelle G représente un atome d'azote et R2 est absent.

Un dixième sous-groupe de composés de l'invention est constitué par les composés de formule (I) dans laquelle R3 représente -C(O)-NH₂.

Les sous-groupes définis ci-dessus pris séparément ou en combinaison font également partie de l'invention.

Parmi les composés de formule (I) selon l'invention, on peut mentionner :
Ex1 : 3-[4-(4-Methanesulfonyl-piperazin-1-yl)-phenyl]-8-aza-bicyclo[3.2.1]octane-8-carboxylic acid (trans-5-hydroxy-adamantan-2-yl)-amide
Ex 2 : 3-[4-(Pyridin-2-yloxy)-phenyl]-8-aza-bicyclo[3.2.1]octane-8-carboxylic acid (trans-5-hydroxy-adamantan-2-yl)-amide
Ex3 : endo-3-Hydroxy-3-[4-(4-methanesulfonyl-piperazin-1-yl)-phenyl]-8-aza-bicyclo[3.2.1]octane-8-carboxylic acid (trans-5-hydroxy-adamantan-2-yl)-amide
Ex 4 : endo-3-Hydroxy-3-[4-(4-methanesulfonyl-piperazin-1-yl)-phenyl]-8-aza-bicyGo[3.2.1]octane-8-carboxylic acid (trans-5-carbamoyl-adamantan-2-yl)-amide
Ex5 : exo-3-[4-(4-Methanesulfonyl-piperazin-1-yl)-phenoxy]-8-aza-bicyclo[3.2.1]octane-8-carboxylic acid (trans-5-hydroxy-adamantan-2-yl)-amide
Ex6 : exo-3-[4-(4-Methanesulfonyl-piperazin-1-yl)-phenoxy]-8-aza-bicyclo[3.2.1]octane-8-carboxylic acid (trans-5-carbamoyl-adamantan-2-yl)-amide
Ex7 : endo-3-[4-(4-Methanesulfonyl-piperazin-1-yl)-phenoxy]-8-aza-bicyclo[3.2.1]octane-8-carboxylic acid (trans-5-hydroxy-adamantan-2-yl)-amide
Ex8 : exo-3-(4-Morpholin-4-yl-phenoxy)-8-aza-bicyclo[3.2.1]octane-8-carboxylic acid (trans-5-carbamoyl-adamantan-2-yl)-amide
Ex9 : exo-3-[5-(4-Isopropoxy-phenyl)-pyridin-2-yloxy]-8-aza-bicyclo[3.2.1]octane-8-carboxylic acid (trans-5-carbamoyl-adamantan-2-yl)-amide
Ex10 : exo-3-[5-(4-Methanesulfonyl-piperazin-1-yl)-pyridin-2-yloxy]-8-aza-bicyclo[3.2.1]octane-8-carboxylic acid (trans-5-carbamoyl-adamantan-2-yl)-amide
Ex11 : exo-3-[5-(4-Methoxy-phenyl)-pyrimidin-2-yloxy]-8-aza-bicyclo[3.2.1]octane-8-carboxylic acid (trans-5-hydroxy-adamantan-2-yl)-amide
Ex12: exo-3-[5-(4-Methoxy-phenyl)-pyrimidin-2-yloxy]-8-aza-bicyclo[3.2.1]octane-8-carboxylic acid (trans-5-carbamoyl-adamantan-2-yl)-amide
Ex13: exo-3-[4-(4-Methanesulfonyl-piperazin-1-yl)-phenoxymethyl]-8-aza-bicyclo[3.2.1]octane-8-carboxylic acid (trans-5-hydroxy-adamantan-2-yl)-amide
Ex14 : exo-3-[4-(4-Methanesulfonyl-piperazin-1-yl)-phenoxymethyl]-8-aza-bicyclo[3.2.1]octane-8-carboxylic acid (trans-5-carbamoyl-adamantan-2-yl)-amide
Ex15 : endo-Hydroxy-3-[4-(4-methanesulfonyl-piperazin-1-yl)-phenoxymethyl]-8-aza-bicyclo[3.2.1]octane-8-carboxylic acid (trans-5-hydroxy-adamantan-2-yl)-amide
Ex16 : endo-3-Hydroxy-3-[4-(4-methanesulfonyl-piperazin-1-yl)-phenoxymethyl]-8-aza-bicyclo[3.2.1]octane-8-carboxylic acid (trans-5-carbamoyl-adamantan-2-yl)-amide
Ex17 : endo-3-[4-(4-Methanesulfonyl-piperazin-1-yl)-phenoxymethyl]-8-aza-bicyclo[3.2.1]octane-8-carboxylic acid (trans-5-hydroxy-adamantan-2-yl)-amide
Ex18 : endo-3-[5-(4-Methanesulfonyl-piperazin-1-yl)-pyridin-2-yloxymethyl]-8-aza-bicyclo[3.2.1]octane-8-carboxylic acid (trans-5-hydroxy-adamantan-2-yl)-amide
Ex19 : endo-3-[5-(4-Methanesulfonyl-piperazin-1-yl)-pyridin-2-yloxymethyl]-8-aza-bicyclo[3.2.1]octane-8-carboxylic acid (trans-5-carbamoyl-adamantan-2-yl)-amide
Ex20 : endo-3-[5-(4-tert-Butyl-piperazin-1-yl)-pyridin-2-yloxymethyl]-8-aza-bicyclo[3.2.1]octane-8-carboxylic acid (trans-5-carbamoyl-adamantan-2-yl)-amide
Ex21 : endo-3-[5-(4-tert-Butyl-piperazin-1-yl)-pyridin-2-yloxymethyl]-8-aza-bicyclo[3.2.1]octane-8-carboxylic acid (trans-5-hydroxy-adamantan-2-yl)-amide
Ex22 : exo-3-[5-(4-Methanesulfonyl-piperazin-1-yl)-pyridin-2-yloxymethyl]-8-aza-bicyclo[3.2.1]octane-8-carboxylic acid (trans-5-carbamoyl-adamantan-2-yl)-amide
Ex23 : exo-3-[5-(4-tert-Butyl-piperazin-1-yl)-pyridin-2-yloxymethyl]-8-aza-bicyclo[3.2.1]octane-8-carboxylic acid (trans-5-hydroxy-adamantan-2-yl)-amide
Ex24 : exo-3-[5-(4-tert-Butyl-piperazin-1-yl)-pyridin-2-yloxymethyl]-8-aza-bicyclo[3.2.1]octane-8-carboxylic acid (trans-5-carbamoyl-adamantan-2-yl)-amide
Ex25 : 3-[5-(4-Methanesulfonyl-piperazin-1-yl)-pyridin-2-yl]-3,8-diaza-bicyclo[3.2.1]octane-8-carboxylic acid (trans-5-hydroxy-adamantan-2-yl)-amide
Ex26 : 3-[5-(4-Methanesulfonyl-piperazin-1-yl)-pyridin-2-yl]-3,8-diaza-bicyclo[3.2.1]octane-8-carboxylic acid (trans-5-carbamoyl-adamantan-2-yl)-amide
Ex27 : exo-3-[5-(4-Trimethylsilanyl-phenyl)-pyridin-2-yloxy]-8-aza-bicyclo[3.2.1]octane-8-carboxylic acid (5-carbamoyl-adamantan-2-yl)-amide

Dans ce qui suit, on entend par groupe protecteur (PG) un groupe qui permet, d'une part, de protéger une fonction réactive telle qu'un hydroxy ou une amine pendant une synthèse et, d'autre part, de régénérer la fonction réactive intacte en fin de synthèse. Des exemples de groupes protecteurs ainsi que des méthodes de protection et de déprotection sont données dans « Protective Groups in Organic Synthesis », Green et al., 3rd Edition (John Wiley & Sons, Inc., New York).

On entend par groupe partant (Lg), dans ce qui suit, un groupe lié à une molécule ou un composé par une liaison et qui peut être facilement clivé de ladite molécule ou dudit composé par rupture hétérolytique de ladite liaison, avec départ d'une paire électronique. Ce groupe peut ainsi être remplacé facilement par un autre groupe lors d'une réaction de substitution, par exemple. De tels groupes partants sont, par exemple, les halogènes ou un groupe hydroxy activé tel qu'un mésyle, tosyle, triflate, acétyle, etc. Des exemples de groupes partants ainsi que des références pour leur préparation sont donnés dans « Advances in Organic Chemistry », J. March, 3rd Edition, Wiley Interscience, p. 310-316.

Conformément à l'invention, on peut préparer les composés de formule générale (I) selon le procédé qui suit.

### Schéma 1

Les composés (I) décrits dans l'invention peuvent être synthétisés en suivant la procédure générale suivante décrite dans le schéma 1 :

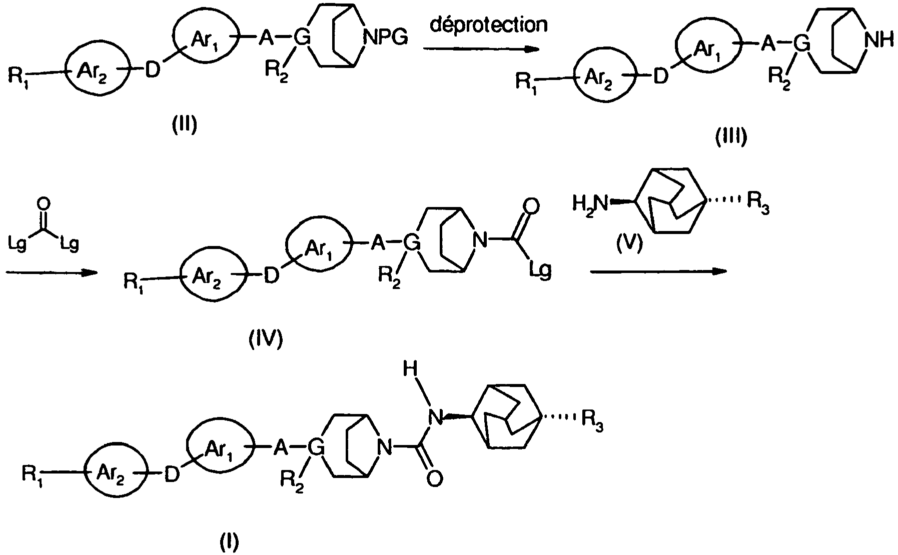

La fonction amine des composés (II) est déprotégée comme décrit dans Green, T.W., et Wutz P.G.M., Protective groups in organic synthesis (1999*)* dans des conditions acides telles que HCl dans le dioxane ou l'acide trifluoacétique dans un solvant tel que le dichlorométhane pour cliver un groupement t-butoxycarbonyle. Le Pd/C sous d'hydrogène ou en présence de formiate d'ammonium dans un solvant tel que le methanol peut être utilisé pour cliver un groupement benzyle. Le 1-chloroethylchloroformiate dans un solvant tel que le dichloroéthane peut être employé pour cliver un groupement méthyle. Les composés (III) obtenus réagissent avec un carbonyle présentant deux groupes partant Lg (par exemple un atome de chlore, un groupe trichlorométhoxy, un groupe para-nitrophényle, un groupe imidazole ou méthyl-imidazolium) en présence d'une base comme la triéthylamine ou le bicarbonate de sodium, dans un solvant tel que le dichlométhane ou le tétrahydrofurane et à une température variant de la température ambiante à 80°C. Les composés de formule (I) sont ensuite obtenus par couplage entre les dérivés activés (IV) et les amines (V) en présence ou non d'une base comme la triéthylamine ou le carbonate de potassium, dans un solvant tel qu'un solvant polaire, par exemple le tétrahydrofurane, le dichlorométhane, l'acétonitrile, le diméthylformamide ou l'eau, à une température variant de la température ambiante à 100°C.

Les hétérocycles de formule générale (V) sont disponibles commercialement ou peuvent être préparés par des méthodes décrites dans la littérature (par exemple WO 2007/077949, US 2005/0215784 A1, US 2005/0245745 A1).

Schéma 2 : Les composés (II) pour lesquels A est une liaison, G est -C-, R₂ est -H, et D est une liaison ou -0 sont appellés ci-après composés (IIa) et peuvent être synthétisés selon le schéma 2 ci-dessous :

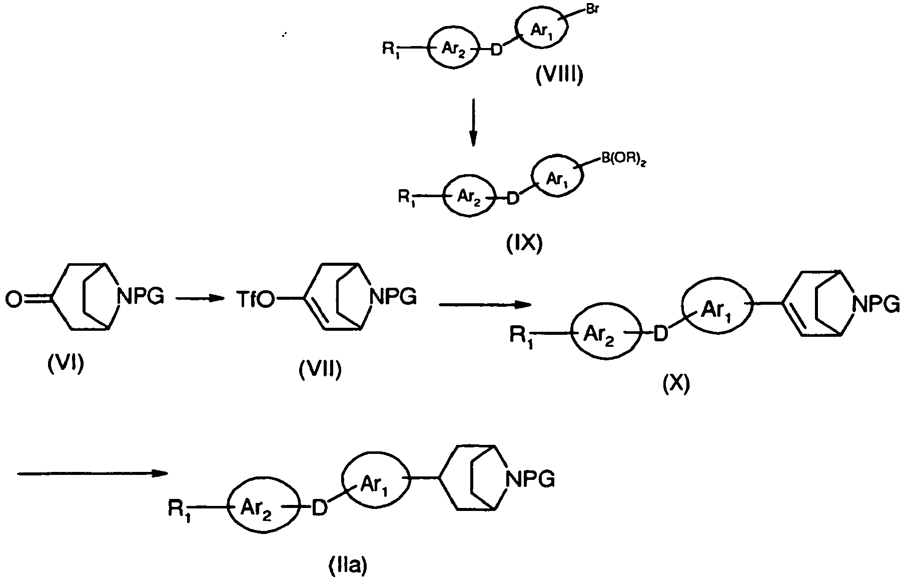

La tropanone protégée sur l'azote (VI) commerciale peut conduire par réaction avec une base forte telle que le lithium bis(trimethylsilyl)amide dans un solvant tel que le THF ou le DME à une température variant de -78°C à température ambiante au vinyl triflate correspondant (VII). Les composés (X) peuvent être obtenus par un couplage de Suzuki en présence d'un catalyseur organométallique tel qu'un dérivé du palladium, tel que le tetrakistriphenylphosphine palladium et d'une base telle que Na₂CO₃ ou Cs₂CO₃ ou le fluorure de potassium dans un solvant tel que le toluène, le DME ou le THF, à des températures variant de la température ambiante à 115°C entre le composé (VII) et le composé (IX). Les composés (IX) peuvent être obtenus par couplage organométallique entre le composé (VIII) et le bis(pinacolato)diboron en présence d'un catalyseur tel qu'un dérivé du palladium, tel que le dichlorodiphenylphosphinoferrocene palladium et d'une base telle que l'acétate de potassium dans un solvant tel le DME ou le THF à des températures variant de la température ambiante à 90°C. La réduction de la double liaison des composés (X) peut être réalisée en présence d'un catalyseur tel que le Pd/C en présence d'hydrogène dans un solvant tel que le méthanol ou l'acétate d'éthyle pour donner les composés (IIa).

**Schéma 3a :** Les composés (II) pour lesquels A est une liaison, D est une liaison, G est -C- et R₂ est -OH, Ar2 est un groupe hétérocycloalkyle ou pour lesquels A représente -O-(CH₂)ₙ- (n=0 ou 1), D est une liaison, G est -C- et R₂ est H, Ar2 est un groupe hétérocycloalkyle, ou pour lesquels A est une liaison, G est un -Net R₂ n'existe pas, Ar2 est un groupe hétérocycloalkyle sont appellés ci-après composés (IIb) et peuvent être obtenus selon le schéma 3a ci-dessous :

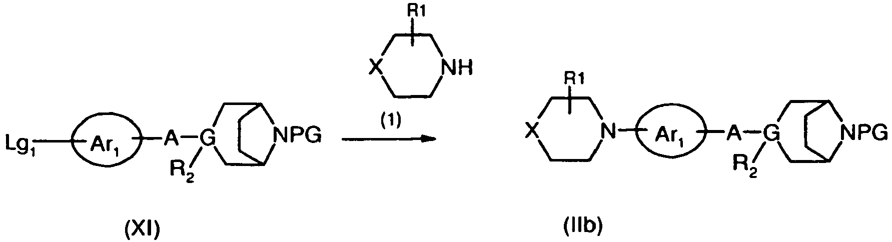

Les composés (XI) où Lg1 représente un groupe partant tel qu'un atome de brome ou d'iode ou un triflate peuvent être engagés dans une réaction de couplage organométallique comme l'amination de Buchwald-Hartwig en présence d'une amine secondaire (1) et de catalyseurs dérivés du palladium tels que le trisdibenzilidene acetone dipalladium ou le 2'-(dimethylamino)-2-biphenylyl-palladium(II) chloride dinorbornylphosphine complex, d'un ligand tel que le S-Phos et d'une base telle que le t-butylate de sodium ou le phosphate de potassium dans un solvant ou mélange de solvants tel que le dioxane, le toluène, le tétrahydrofurane ou le DME, à des températures variant de de la température ambiante à 115°C.

**Schéma 3b :** Les composés (II) pour lesquels A est une liaison, D est une liaison, G est -C- et R₂ est -OH, Ar2 est un aryle ou hétéroaryle ou pour lesquels A représente -O-(CH₂)ₙ- (n=0 ou 1), D est une liaison, G est -C- et R₂ est H, Ar2 est un aryle ou hétéroaryle ou pour lesquels A est une liaison, G est un -N- et R₂ n'existe pas, Ar2 est un aryle ou hétéroaryle sont appellées ci-après composés (IIb') et peuvent être obtenus selon le schéma 3b ci-dessous :

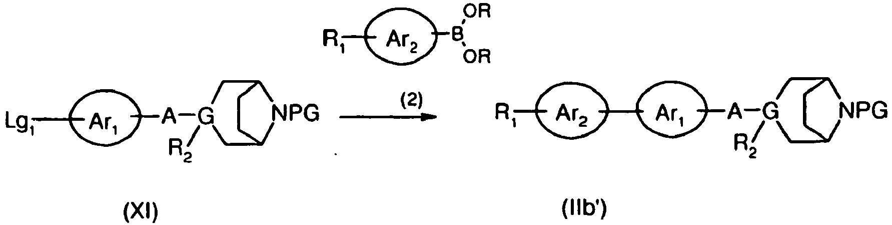

Les composés (XI) où Lg1 représente un groupe partant tel qu'un atome de brome ou d'iode ou un triflate peuvent être engagés dans une réaction de couplage organométallique comme le couplage de Suzuki en présence d'un dérivé boronique (2) (avec R représente un hydrogène ou forme ensemble un -OCMe₂CMe₂O-) et d'un catalyseur organométallique tel qu'un dérivé du palladium en présence ou non d'une phosphine, tel que le tetrakistriphenylphosphine palladium et d'une base telle que Na₂CO₃ , le carbonate de césium ou le fluorure de potassium dans un solvant tel que le toluène, le DME ou le THF, à des températures variant de la température ambiante à 115°C.

**Schéma 4 :** Les composés (XI) pour lesquels A est une liaison, G est -C-, D est une liaison et R₂ est -OH sont appellées ci-après composés (XIa) et peuvent être obtenus selon le schéma 4 ci-dessous :

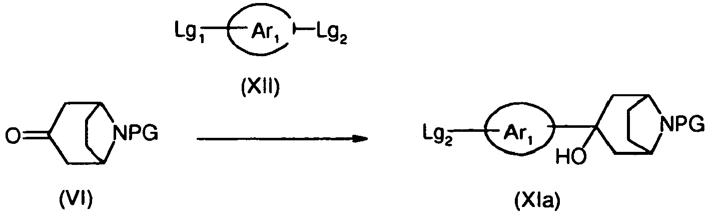

Les composés (XII) où Lg1 et Lg2 représentent un groupe partant tel qu'un atome d'halogène par exemple un atome de brome et Ar1 représente un noyau aryle ou hétéroaryle peuvent réagir avec une base forte telle que le butyllithium ou le lithium bis(trimethylsilyl)amide dans un solvant tel que le THF ou l'hexane à une température variant entre -78°C et température ambiante pour former le monoanion correspondant. Celui-ci peut conduire en présence de la tropanone (VI) commerciale dans un solvant tel que le THF ou l'hexane à une température variant entre -78°C et température ambiante aux composés (XIa).

**Schéma 5 :** Les composés (XI) pour lesquels A représente -O-(CH₂)ₙ- (n=0 ou 1), G est -C-, R₂ est -H sont appellées ci-après composés (Xlb) et peuvent être obtenus selon le schéma 5 suivant :

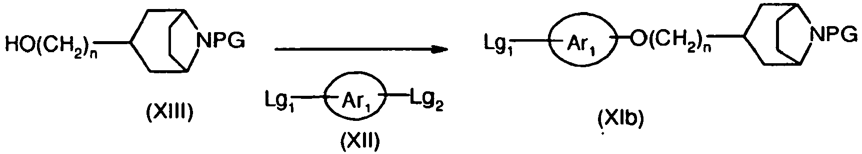

Les composés (XIII) peuvent réagir avec le composé (XII) où Lg2 représente un groupe partant tel qu'un atome de fluor et Ar1 représente un noyau aryle ou hétéroaryle en présence d'une base forte telle que l'hydrure de sodium ou le t-butoxide de potassium dans un solvant que tel que la NMP ou le DMF à des températures variant de 0°à 130°C pour conduire aux composés (Xlb).

**Schéma 6 :** Les composés (XI) pour lesquels A est une liaison, G est -N- , R2 est absent sont appellées ci-après composés (XIc) et peuvent être obtenus selon le schéma 6 ci-dessous :

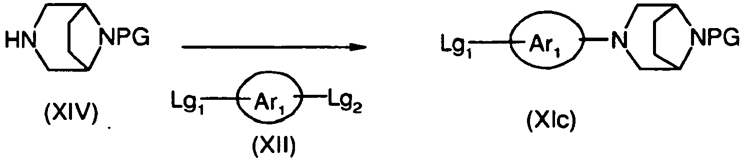

Le 3,8-Diaza-bicyclo[3.2.1]octane (XIV) protégé sur la position 8 peut conduire aux composés (Xlc) en présence des composés (XII) où Lg2 représente un groupe partant tel qu'un atome de brome et Ar1 représente un noyau aryle ou hétéroaryle par une réaction d'amination de Buchwald-Hartwig telle que décrite ci-dessus.

**Schéma 7:** Alternativement, les composés (II) pour lesquels A représente -0-(CH₂)ₙ- (n=0 ou 1), G est -C-, R₂ est -H et D est une liaison sont appellées ci-après composés (IIc) et peuvent être obtenus selon le schéma 7 ci-dessous :

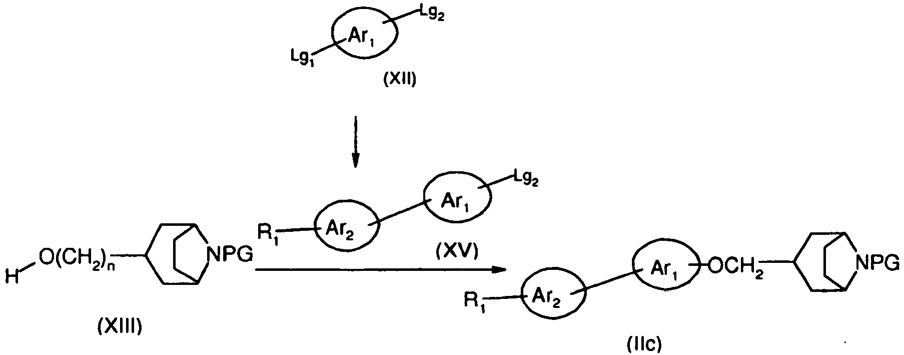

Les composés (XIII) peuvent réagir avec les composés (XV) où Lg2 représente un groupe partant tel qu'un atome d'halogène tel qu'un atome de fluor en présence d'une base forte telle que l'hydrure de sodium ou le tert-butylate de potassium dans un solvant que le DMF ou la NMP à des températures variant de 0°C à 130°C pour conduire au composé (IIc). Les composés (XV) peuvent être disponibles commercialement ou synthétisés par des réactions telles que l'amination de Buchwald-Hartwig dans des conditions telles que décrites ci-dessus.

**Schéma 8 :** Les composés (II) pour lesquels A est un -O-CH₂-, G est -C-, R₂ est -OH et D est une liaison sont appellées ci-après composés (IId) et peuvent être obtenus selon le schéma 8 ci-dessous :

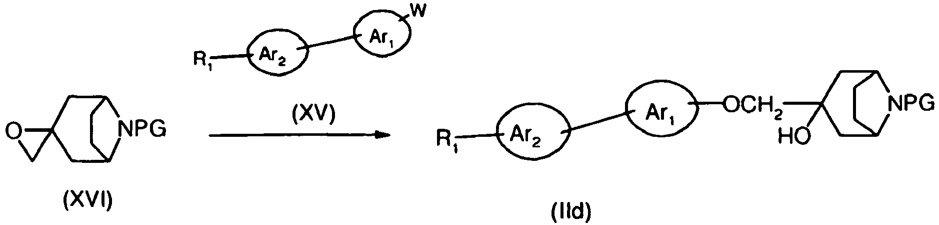

Les composés (XVI) obtenus selon la procédure décrite dans la littérature (J. Het. Chem. (1968), pp467*)* peuvent réagir avec les composés (XV) où W est un groupe OH en présence d'une base forte telle que l'hydrure de sodium ou la soude dans un solvant polaire tel que le DMF, le diméthylsulfoxyde ou l'eau à une température variant de 0°C à 130°C pour conduire aux composés (IId).

Dans les schémas 1 à 8, les composés de départ et les réactifs, quand leur mode de préparation n'est pas décrit, sont disponibles dans le commerce ou décrits dans la littérature, ou bien peuvent être préparés selon des méthodes qui y sont décrites ou qui sont connues de l'Homme du métier.

L'invention, selon un autre de ses aspects, a également pour objet les composés de formules (IX), (X), (XI), (XIa), (Xlb), (Xlc), (II), (IIa), (IIb), (IIb'), (IIc), (IId), (III), (IV). Ces composés sont utiles comme intermédiaires de synthèse des composés de formule (I).

Les exemples suivants décrivent la préparation de certains composés conformes à l'invention. Ces exemples ne sont pas limitatifs et ne font qu'illustrer la présente invention. Les numéros des composés exemplifiés renvoient à ceux donnés dans le tableau ci-après, qui illustre les structures chimiques et les propriétés physiques de quelques composés selon l'invention.

Les abréviations et formules brutes suivantes sont utilisées :
- °C: Degré Celsius
- Cs₂CO₃: carbonate de césium
- DIAD: 1,1'-(azodicarbonyl)dipiperidine
- DME: diméthoxyéthane
- DMF: diméthylformamide
- DMSO: diméthyl sulfoxide
- h: heure(s)
- HCl: acide chlorhydrique
- K₂CO₃: Carbonate de potassium
- KHSO₄: bisulfate de potassium
- LC/MS: chromatographie liquide/ spectrométrie de masse
- M: molaire
- MgSO₄: Sulfate de magnésium
- MHz: MegaHertz
- min: minute(s)
- mL: millilitre(s)
- mmol: millimole(s)
- N: normal
- Na₂CO₃: Carbonate de sodium
- NaHCO₃: Hydrogénocarbonate de sodium
- NMP: N-méthylmorpholine
- P₂O₅: phosphorus pentoxide
- ppm: parties par millions
- psi: pound per square inch
- S-Phos: 2-dicyclohexylphosphino-2',6'-dimethoxybiphenyl
- tBu: *tert*-butyl
- XantPhos: 9,9-dimethyl-4,5-bis(diphenylphosphino)xanthene

### EXEMPLE 1 :

### 3-[4-(4-Methanesulfonyl-piperazin-1-yl)-phenyl]-8-aza-bicyclo[3.2.1]octane-8-carboxylic acid (trans-5-hydroxy-adamantan-2-yl)-amide-(Composé n°1)

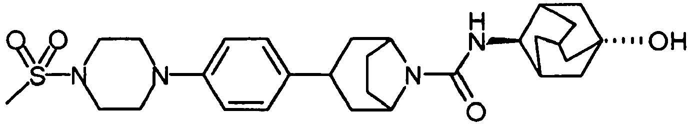

### 1.1/ 3-Trifluoromethanesulfonyloxy-8-aza-bicyclo[3.2.1]oct-2-ene-8-carboxylic acid tert-butyl ester

On place le 3-oxo-8-aza-bicyclo[3.2.1]octane-8-carboxylic acid tert-butyl ester (5 g, 22,2 mmol) dans 24 mL de THF anhydre et on refroidit la solution à -70°C sous azote. On ajoute goutte à goutte le lithium bis(trimethylsilyl)amide 1N dans le THF (24,4 mL, 24,4 mmol). Après 45 min d'agitation à -70°C, on ajoute goutte à goutte le N-phenyltrifluoromethanesulphonimide (8,7 g, 24,4 mmol) placé dans 25 mL de THF anhydre. On laisse remonter lentement la température du milieu réactionnel. L'agitation est maintenue 16h à température ambiante. Après concentration à sec, le brut obtenu est chromatographié sur gel de silice en éluant avec un mélange cyclohexane/éther (90/10). On obtient 10,2 g de 3-trifluoromethanesulfonyloxy-8-aza-bicyclo[3.2.1]oct-2-ene-8-carboxylic acid tert-butyl ester attendu.
[M+H⁺] = 258 (-OtBu)

### 1.2/ 1-(4-Bromo-phenyl)-4-methanesulfonyl-piperazine

On place la 1-(4-bromo-phenyl)-piperazine (5 g, 20,7 mmol) dans 104 mL de dichlorométhane puis on ajoute la triéthylamine (4,34 mL, 31,1 mmol) suivie goutte à goutte du chlorure de méthane sulfonyle (1,93 mL, 24,9 mmol). L'agitation est maintenue 16h. Après addition de 30 mL d'eau et agitation, la phase organique est lavée avec une solution aqueuse saturée en chlorure de sodium, séchée sur MgSO₄ et concentrée. On obtient 6,6 g de 1-(4-bromo-phenyl)-4-methanesulfonyl-piperazine attendu.
[M+H⁺] = 319

### 1.3/ 1-Methanesulfonyl-4-[4-(4,4,5,5-tetramethyl-[1,3,2]dioxaborolan-2-yl)-phenyl]-piperazine

On place la 1-(4-bromo-phenyl)-4-methanesulfonyl-piperazine (2 g, 6,3 mmol) dans 42 mL de DME. On ajoute le bis(pinacolato)diboron (1,91 g, 7,52 mmol), l'acétate de potassium (1,85 g, 18,8 mmol) et le dichlorodiphenylphosphinoferrocene palladium (0,511 g, 0,63 mmol). Le milieu réactionnel est chauffé au reflux pendant 16h. Après hydrolyse et extraction à l'acétate d'éthyle, la phase organique est filtrée sur Célite®, lavée avec une solution aqueuse saturée en chlorure de sodium, séchée sur MgSO₄ et concentrée à sec. Le brut obtenu est chromatographié sur gel de silice en éluant avec un mélange dichlorométhane/acétate d'éthyle (95/5). On obtient 1,8 g de 1-methanesulfonyl-4-[4-(4,4,5,5-tetramethyl-[1,3,2]dioxaborolan-2-yl)-phenyl]-piperazine.
[M+H⁺] = 367

### 1.4/ 3-[4-(4-Methanesulfonyl-piperazin-1-yl)-phenyl]-8-aza-bicyclo[3.2.1]oct-2-ene-8-carboxylic acid tert-butyl ester

On place la 1-methanesulfonyl-4-[4-(4,4,5,5-tetramethyl-[1,3,2]dioxaborolan-2-yl)-phenyl]-piperazine (1,8 g, 4,9 mmol), 3-trifluoromethanesulfonyloxy-8-aza-bicyclo[3.2.1]oct-2-ene-8-carboxylic acid tert-butyl ester (3,8 g, 5,9 mmol) dans 35 mL de DME et 6,14 mL d'une solution aqueuse 2N de K₂CO₃. Après dégazage à l'azote, on ajoute le tetrakistriphenylphosphine palladium (1,136 g, 0, 98 mmol). Le milieu réactionnel est chauffé au reflux pendant 6h. Après hydrolyse et addition d'acétate d'éthyle, le milieu réactionnel est filtré sur Célite®. La phase aqueuse est ensuite extraite à l'acétate d'éthyle. La phase organique est lavée avec une solution aqueuse saturée en NaHCO₃ puis à l'eau. Après séchage sur MgSO₄ et concentration à sec, le brut obtenu est chromatographié sur gel de silice en éluant avec un gradient d'acétate d'éthyle dans le dichlorométhane variant de 0% à 20%. On obtient 0,83 g de 3-[4-(4-methanesulfonyl-piperazin-1-yl)-phenyl]-8-aza-bicyclo[3.2.1]oct-2-ene-8-carboxylic acid tert-butyl ester.
[M+H⁺] = 448

### 1.5/ 3-[4-(4-Methanesulfonyl-piperazin-1-yl)-phenyl]-8-aza-bicyclo[3.2.1]octane-8-carboxylic acid tert-butyl ester

On solubilise le 3-[4-(4-methanesulfonyl-piperazin-1-yl)-phenyl]-8-aza-bicyclo[3.2.1]oct-2-ene-8-carboxylic acid tert-butyl ester (0,83 g, 1,85 mmol) dans 93 mL de methoxyéthanol. Puis on ajoute le palladium sur charbon 10% (0,395 g). Le milieu réactionnel est agité 5h sous 30 psi d'hydrogène à 25°C. Après filtration sur Célite® et concentration, on obtient 0,76 g de 3-[4-(4-methanesulfonyl-piperazin-1-yl)-phenyl]-8-aza-bicyclo[3.2.1]octane-8-carboxylic acid tert-butyl ester attendu.
[M+H⁺] = 450

### 1.6/ 3-[4-(4-Methanesulfonyl-piperazin-1-yl)-phenyl]-8-aza-bicyclo[3.2.1]octane

On solubilise le 3-[4-(4-methanesulfonyl-piperazin-1-yl)-phenyl]-8-aza-bicyclo[3.2.1]octane-8-carboxylic acid tert-butyl ester (0,758 g, 1,69 mmol) dans 158 mL de dichlorométhane. Puis on ajoute HCl 4N dans le dioxane (4,2 mL, 16,8 mmol). Après agitation 16h à température ambiante, le milieu réactionnel est concentré, repris à l'eau, lavé avec de l'acétate d'éthyle. Le pH de la phase aqueuse est ensuite ajusté à 10 avec une solution aqueuse 5N de soude. Après extraction au dichlorométhane, lavage à l'eau puis avec une solution aqueuse saturée en chlorure de sodium, la phase organique est séchée sur MgSO₄ puis concentrée à sec. On obtient 0,51 g de 3-[4-(4-methanesulfonyl-piperazin-1-yl)-phenyl]-8-aza-bicyclo[3.2.1]octane attendu utilisé tel quel par la suite.
[M+H⁺] = 350

### 1.7/ 3-[4-(4-Methanesulfonyl-piperazin-1-yl)-phenyl]-8-aza-bicyclo[3.2.1]octane-8-carboxylic acid (trans-5-hydroxy-adamantan-2-yl)-amide

On solubilise le 3-[4-(4-methanesulfonyl-piperazin-1-yl)-phenyl]-8-aza-bicyclo[3.2.1]octane (0,26 g , 0,74 mmol) dans 10 mL de dichlorométhane. On ajoute la diisopropyléthylamine (0,25 mL, 1,49 mmol). Le milieu réactionnel est placé à 0°C sous azote. On ajoute le triphosgène (0,11 g, 0,37 mmol). L'agitation est maintenue 4h à température ambiante. Après avoir additionné à nouveau de la diisopropyléthylamine (0,12 mL, 0,74 mmol) et 10 mL de DMF anhydre, on ajoute le trans-4-amino-adamantan-1-ol (0,137g, 0,82 mmol). Après 24h d'agitation, le milieu réactionnel est chauffé à 50°C pendant 16h. On ajoute alors du dichlorométhane et de l'eau. Après extraction, la phase organique est lavée à l'eau puis avec une solution aqueuse saturée en chlorure de sodium. Elle est séchée sur MgSO₄ puis concentrée à sec. Le brut obtenu est repris à l'acétonitrile et trituré. L'insoluble est rincé au pentane et essoré. On obtient 0,10 g de 3-[4-(4-methanesulfonyl-piperazin-1-yl)-phenyl]-8-aza-bicyclo[3.2.1]octane-8-carboxylic acid (trans-5-hydroxy-adamantan-2-yl)-amide attendu.
Point de fusion = 250 °C; [M+H⁺] = 543
**RMN 1H (400 MHz, DMSO-d6) δ ppm** 7,1 (m, 2H), 6,9 (m, 2H), 5,85 (m, 1 H), 4,36 (sl, 1H), 4,3 (sl, 2H), 3,7 (m, 1H), 3,3 to 3,15 (m, 8H), 3,0 (m, 1H), 2,9 (s, 3H), 2,5-2,25 (m, 2H), 2,1-1,25 (m, 19H).

### EXEMPLE 2 :

### 3-[4-(Pyridin-2-yloxy)-phenyl]-8-aza-bicyclo[3.2.1]octane-8-carboxylic acid (trans-5-hydroxy-adamantan-2-yl)-amide (Composé n°2)

### 2.1/ 2-(4-bromo-phenoxy)-pyridine

On place le 4-bromophénol (2 g, 11,5 mmol) dans 10 mL de DMF anhydre sous azote. On ajoute l'hydrure de sodium (0.51 g, 12, 7 mmol). Après 20 min d'agitation à température ambiante, on ajoute la 2-fluoropyridine (1,05 mL, 12,1 mmol) et le milieu réactionnel est chauffé à 105°C pendant 7h. Après hydrolyse, le pH est ajusté à 8 avec une solution aqueuse de HCl 1 N. Le milieu réactionnel est ensuite extrait à l'acétate d'éthyle. La phase organique est lavée avec une solution aqueuse saturée en chlorure de sodium, séchée sur MgSO₄ et concentrée à sec. Le brut obtenu est chromatographié sur gel de silice en éluant avec un gradient d'acétate d'éthyle dans l'heptane variant de 0% à 30%. On obtient 2,22 g de 2-(4-bromo-phenoxy)-pyridine attendu.
[M+H⁺] = 250

### 2.2/ 2-[4-(4,4,5,5-Tetramethyl-[1,3,2]dioxaborolan-2-yl)-phenoxy]-pyridine

Suivant un mode opératoire identique à celui décrit à l'étape 1.3, à partir de 2-(4-bromo-phenoxy)-pyridine (1,2g, 4,8 mmol), on obtient 0,63 g de 2-[4-(4,4,5,5-tetramethyl-[1,3,2]dioxaborolan-2-yl)-phenoxy]-pyridine attendu après chromatographie sur gel de silice en éluant le brut obtenu avec un gradient d'acétate d'éthyle dans le dichlorométhane variant de 0% à 4%.
[M+H⁺] = 297

### 2.3/ 3-[4-(Pyridin-2-yloxy)-phenyl]-8-aza-bicyclo[3.2.1]oct-2-ene-8-carboxylic acid tert-butyl ester

On place la 2-[4-(4,4,5,5-tetramethyl-[1,3,2]dioxaborolan-2-yl)-phenoxy]-pyridine (0,62 g, 2,1 mmol), 3-trifluoromethanesulfonyloxy-8-aza-bicyclo[3.2.1]oct-2-ene-8-carboxylic acid tert-butyl ester (0,75 g, 2,1 mmol) (intermédiaire 1), le tetrakistriphenylphosphine palladium (0,24 g, 0,21 mmol) et du chlorure de lithium (0,107 g, 2,52 mmol) dans 10 mL de DME et 2,6 mL d'une solution aqueuse 2N de K₂CO₃. Le milieu réactionnel est chauffé à reflux pendant 16h. Après hydrolyse et extraction à l'acétate d'éthyle, la phase organique est séchée sur MgSO₄ et concentrées à sec. Le brut obtenu est chromatographié sur gel de silice en éluant avec un gradient d'acétate d'éthyle dans l'heptane variant de 0% à 30%. On obtient 0,45 g de 3-[4-(pyridin-2-yloxy)-phenyl]-8-aza-bicyclo[3.2.1]oct-2-ene-8-carboxylic acid tert-butyl ester attendu.
[M+H⁺] = 379

### 2.4/ 3-[4-(Pyridin-2-yloxy)-phenyl]-8-aza-bicyclo[3.2.1]octane-8-carboxylic acid tert-butyl ester

Suivant un mode opératoire identique à celui décrit à l'étape 1.5, à partir de 3-[4-(pyridin-2-yloxy)-phenyl]-8-aza-bicyclo[3.2.1]oct-2-ene-8-carboxylic acid tert-butyl ester (0,45 g, 0,89 mmol), on obtient 0,36 g de 3-[4-(pyridin-2-yloxy)-phenyl]-8-aza-bicyclo[3.2.1]octane-8-carboxylic acid tert-butyl ester attendu en chromatographiant sur gel de silice en éluant le brut obtenu avec gradient de méthanol dans le dichlorométhane variant de 0% à 2%.
[M+H⁺] = 381

### 2.5/ 3-[4-(Pyridin-2-yloxy)-phenyl]-8-aza-bicyclo[3.2.1]octane

Suivant un mode opératoire identique à celui décrit à l'étape 1.6, à partir de 3-[4-(pyridin-2-yloxy)-phenyl]-8-aza-bicyclo[3.2.1]octane-8-carboxylic acid tert-butyl ester (0,34 g, 0,9 mmol), on obtient 0,235 g de 3-[4-(pyridin-2-yloxy)-phenyl]-8-aza-bicyclo[3.2.1]octane attendu après concentration à sec et traitement avec une solution aqueuse saturée en NaHCO₃, extraction à l'acétate d'éthyle, lavage avec une solution aqueuse saturée en chlorure de sodium, séchage sur MgSO₄ et concentration à sec.
[M+H⁺] = 281

### 2.6/ 3-[4-(Pyridin-2-yloxy)-phenyl]-8-aza-bicyclo[3.2.1]octane-8-carboxylic acid (trans-5-hydroxy-adamantan-2-yl)-amide

Suivant un mode opératoire identique à celui décrit à l'étape 1.7, à partir de 3-[4-(pyridin-2-yloxy)-phenyl]-8-aza-bicyclo[3.2.1]octane (0,24g, 0,84 mmol), on obtient 0,054 g de 3-[4-(pyridin-2-yloxy)-phenyl]-8-aza-bicyclo[3.2.1]octane-8-carboxylic acid (trans-5-hydroxy-adamantan-2-yl)-amide attendu en chromatographiant sur gel de silice le brut obtenu avec comme éluant un gradient de méthanol/ammoniaque dans le dichlorométhane variant de 100/0/0 à 9/1/0,1 (dichlorométhane/méthanol/ammoniaque).
Point de fusion = 103°C; [M+H⁺] = 474
**RMN 1H (400 MHz, DMSO-d6) δ ppm** 8,15 (m, 1H), 7,85 (m, 1H), 7,25 (m, 1 H), 7,2 (m, 1 H), 7,1 (m, 1 H), 7,05-6,95 (m, 4H), 5,85 (dd, J = 18 et 6 Hz, 1 H), 4,35 (m, 3H), 3,7 (m, 1 H), 3,15 (m, 1 H), 2,4 (m, 1 H), 2,1- 1,15 (m, 19H)

### EXEMPLE 3 :

### endo-3-Hydroxy-3-[4-(4-methanesulfonyl-piperazin-1-yl)-phenyl]-8-aza-bicyclo[3.2.1]octane-8-carboxylic acid (trans-5-hydroxy-adamantan-2-yl)-amide

### (Composé n°3)

### 3.1/ 8-Benzyl-3-(4-bromo-phenyl)-8-aza-bicyclo[3.2.1]octan-endo-3-ol

On place le dibromobenzène (1,09 g, 4,64 mmol) dans 8 mL de THF anhydre sous azote à -70°C. On ajoute le n-butyllithium 2,5M dans hexane (1,86 mL, 4,64 mmol). L'agitation est maintenue 1h à -70°C. On ajoute ensuite lentement la 8-benzyl-8-aza-bicyclo[3.2.1]octan-3-one (0,5 g, 2,32 mmol) dissoute dans 2 mL de THF anhydre. Après 1h d'agitation à -70°C, on laisse remonter le milieu réactionnel à température ambiante et l'agitation est maintenue 16h. Après hydrolyse et extraction du milieu réactionnel à l'acétate d'éthyle, la phase organique est séchée sur MgSO₄. Le brut obtenu est chromatographié sur gel de silice en éluant avec un gradient de méthanol dans le dichlorométhane variant de 0% à 10%. On obtient 0,53 g de 8-benzyl-3-(4-bromo-phenyl)-8-aza-bicyclo[3.2.1]octan-endo-3-ol attendu.
[M+H⁺] = 372

### 3.2/ 8-Benzyl-3-[4-(4-methanesulfonyl-piperazin-1-yl)-phenyl]-8-aza-bicyclo[3.2.1]octan-endo-3-ol

On place le 8-benzyl-3-(4-bromo-phenyl)-8-aza-bicyclo[3.2.1]octan-endo-3-ol (1,73 g, 4,66 mmol) dans 23 mL de DME sous azote. On ajoute la 1-methanesulfonyl-piperazine (0,92 g, 5,6 mmol) et le catalyseur 2'-(dimethylamino)-2-biphenylyl-palladium(II) chloride dinorbornylphosphine complex (0,26 g, 0,47 mmol). On ajoute le lithium bis(trimethylsilyl)amide 1N dans le THF (10,3 mL, 10,3 mmol). Le milieu réactionnel est chauffé au reflux pendant 16h. On rajoute du catalyseur 2'-(dimethylamino)-2-biphenylyl-palladium(11) chloride dinorbornylphosphine complex (0,1 g, 0,18 mmol) et l'agitation au reflux est maintenue 6h supplémentaires. On rajoute alors du lithium bis(trimethylsilyl)amide 1N dans le THF (5,1 mL, 5,1 mmol), du catalyseur 2'-(dimethylamino)-2-biphenylyl-palladium(II) chloride dinorbornylphosphine complex (0,1 g, 0,18 mmol), et de la 1-methanesulfonyl-piperazine (0,5 g, 3,05 mmol). Après chauffage au reflux 16h, on hydrolyse avec une solution aqueuse saturée de NaHCO₃, puis on extrait à l'acétate d'éthyle. La phase organique est lavée avec une solution aqueuse saturée en chlorure de sodium et séchée sur MgSO₄. Le brut obtenu est chromatographié sur gel de silice en éluant avec un gradient de méthanol/ammoniaque dans le dichlorométhane variant de 100/0/0 à 9/1/0,1 (dichlorométhane/méthanol/ammoniaque). On obtient 0,67 g de 8-benzyl-3-[4-(4-methanesulfonyl-piperazin-1-yl)-phenyl]-8-aza-bicyclo[3.2.1]octan-endo-3-ol attendu.
[M+H⁺] = 456

### 3.3/ 3-[4-(4-Methanesulfonyl-piperazin-1-yl)-phenyl]-8-aza-bicyclo[3.2.1]octan-endo-3-ol

On place le 8-benzyl-3-[4-(4-methanesulfonyl-piperazin-1-yl)-phenyl]-8-aza-bicyclo[3.2.1]octan-endo-3-ol (0,67g, 1,46 mmol) dans 15 mL de méthanol. On ajoute Pd/C 10%, ~50% dans l'eau (0,17 g) et du formiate d'ammonium (0,92 g, 14,6 mmol). Le milieu réactionnel est chauffé au reflux pendant 2h. Après filtration du catalyseur, le filtrat est concentré. Le brut obtenu est chromatographié sur gel de silice en éluant avec un gradient de méthanol/ammoniaque dans le dichlorométhane variant de 100/0/0 à 80/20/2 (dichlorométhane/méthanol/ammoniaque). On obtient 0,26 g de 3-[4-(4-methanesulfonyl-piperazin-1-yl)-phenyl]-8-aza-bicyclo[3.2.1]octan-endo-3-ol attendu.
[M+H⁺] = 366

### 3.4/ endo-3-Hydroxy-3-[4-(4-methanesulfonyl-piperazin-1-yl)-phenyl]-8-aza-bicyclo[3.2.1]octane-8-carboxylic acid (trans-5-hydroxy-adamantan-2-yl)-amide

On place le 3-[4-(4-methanesulfonyl-piperazin-1-yl)-phenyl]-8-aza-bicyclo[3.2.1]octan-endo-3-ol (0,25 g, 0,68 mmol) dans 22 mL d'un mélange 50/50 de dichlorométhane/solution aqueuse saturée en NaHCO₃ à 0 °C. On ajoute le phosgène 20% dans le toluène (0,54 mL, 1,5 mmol). Après 1 h20 d'agitation, on rajoute du phosgène 20% dans le toluène (0,16 mL). Après extraction de la phase aqueuse au dichlorométhane, la phase organique est séchée sur MgSO₄. Le brut obtenu est placé dans 5,5 mL de DMF anhydre sous azote. On ajoute la triéthylamine (0,19 mL, 1,38 mmol) suivie du trans-4-amino-adamantan-1-ol (0,14 g, 0,82 mmol) dissous dans 1,5 mL de DMF anhydre. Le milieu réactionnel est chauffé à 50°C pendant 2h30. Après hydrolyse avec une solution aqueuse saturée en NaHCO₃, et extraction à l'acétate d'éthyle, la phase organique est lavée avec une solution aqueuse saturée en chlorure de sodium et séchée sur MgSO₄. Le brut obtenu est chromatographié sur gel de silice en éluant avec un gradient de méthanol dans le dichlorométhane variant de 0% à 10%. On obtient 0,075 g de endo-3-hydroxy-3-[4-(4-methanesulfonyl-piperazin-1-yl)-phenyl]-8-aza-bicyclo[3.2.1]octane-8-carboxylic acid (trans-5-hydroxy-adamantan-2-yl)-amide.
Point de fusion > 250°C; [M+H⁺]= 559
**RMN 1H (400 MHz, DMSO-d6) δ ppm** : 7,15 (d, J=8,6 Hz, 2H), 6,85 (d, J=8,6 Hz, 2H), 5,85 (d, J=5,8 Hz, 1 H), 4,75 (s, 1 H), 4,4 (s, 1 H), 4,25 (m, 2H), 3,7 (m, 1 H), 3,2 (m, 9H), 2,9 (s, 3H), 2,3-1,3 (m, 21 H).

### EXEMPLE 4 :

### endo-3-[4-(4-Methanesulfonyl-piperazin-1-yl)-phenoxy]-8-aza-bicyclo[3.2.1]octane-8-carboxylic acid (trans-5-hydroxy-adamantan-2-yl)-amide

### (Composé n °7)

### 4.1 / endo-3-(4-Bromo-phenoxy)-8-methyl-8-aza-bicyclo[3.2.1]octane

On place l'hydrure de sodium (1,12 g, 46,7 mmol) dans 40 mL de DMF anhydre sous azote. On ajoute lentement la tropine (4,4 g, 31,2 mmol) dissoute dans 20 mL de DMF anhydre. Le milieu réactionnel est chauffé à 65°C pendant 1h puis on ajoute lentement le 4-fluorobenzène placé dans 10 mL de DMF anhydre. Après agitation à 65°C pendant 3h, le milieu est hydrolysé avec un mélange eau/glace. On extrait à l'acétate d'éthyle. La phase organique est rassemblée, lavée à l'eau puis avec une solution aqueuse saturée en chlorure de sodium et séchée sur MgSO₄. Le brut obtenu est repris au toluène et concentré à sec. Après recristallisation dans un mélange pentane/diéthyléther, on obtient 1,87 g de l'endo-3-(4-bromo-phenoxy)-8-methyl-8-aza-bicyclo[3.2.1]octane attendu.
[M+H⁺] = 296

### 4.2/ endo-3-[4-(4-Methanesulfonyl-piperazin-1-yl)-phenoxy]-8-methyl-8-aza-bicyclo[3.2.1]octane

On place l'endo-3-(4-bromo-phenoxy)-8-methyl-8-aza-bicyclo[3.2.1]octane (0,5 g, 1,69 mmol), la 1-methanesulfonyl-piperazine (0,33 g, 2,0 mmol), le catalyseur 2'-(dimethylamino)-2-biphenylyl-palladium(II) chloride dinorbornylphosphine complex (0,095 g, 0,17 mmol), et le phosphate de potassium (0,89 g, 4,22 mmol) dans 8,5 mL de DME sous N₂. Le milieu réactionnel est agité à 90°C pendant 16h. Après hydrolyse et extraction à l'acétate d'éthyle, la phase organique est lavée avec une solution aqueuse saturée en chlorure de sodium et séchée sur MgSO₄. Le brut obtenu est chromatographié sur gel de silice en éluant avec un gradient de méthanol/ammoniaque dans le dichlorométhane variant de 100/0/0 à 90/10/1 (dichlorométhane/ méthanol/ammoniaque). On obtient 0,53 g de l'endo-3-[4-(4-methanesulfonyl-piperazin-1-yl)-phenoxy]-8-methyl-8-aza-bicyclo[3.2.1]octane attendu.
[M+H⁺] = 380

### 4.3/ endo-3-[4-(4-Methanesulfonyl-piperazin-1-yl)-phenoxy]-8-aza-bicyclo[3.2.1]octane

On place l'endo-3-[4-(4-methanesulfonyl-piperazin-1-yl)-phenoxy]-8-methyl-8-aza-bicyclo[3.2.1]octane (0,91 g, 2, 4 mmol) dans 4,8 mL de dichloroéthane à 0°C sous azote. On ajoute le 1-chloroéthyl chloroformate (0,52 mL, 4,8 mmol). Le milieu réactionnel est agité 16h à 80°C. Après concentration à sec, on ajoute 5 mL de méthanol et on chauffe à 80°C pendant 1h30. Après évaporation du solvant, on ajoute une solution aqueuse saturée en NaHCO₃ et on extrait au dichlorométhane. La phase organique est séchée sur MgSO₄. Le brut obtenu est chromatographié sur gel de silice en éluant avec un gradient de méthanol/ammoniaque dans le dichlorométhane variant de 95/5/0,5 à 90/10/1 (dichlorométhane/méthanol/ammoniaque). On obtient 0,42 g d'endo-3-[4-(4-Methanesulfonyl-piperazin-1-yl)-phenoxy]-8-aza-bicyclo[3.2.1]octane attendu.
[M+H⁺] = 366

### 4.4/ endo-3-[4-(4-Methanesulfonyl-piperazin-1-yl)-phenoxy]-8-aza-bicyclo[3.2.1]octane-8-carboxylic acid (trans-5-hydroxy-adamantan-2-yl)-amide

On place l'endo 3-[4-(4-methanesulfonyl-piperazin-1-yl)-phenoxy]-8-aza-bicyclo[3.2.1]octane (0,21 g, 0,57 mmol) dans 6 mL de dichlorométhane à 0°C. Puis on ajoute la triéthylamine (0,18 mL, 1,26 mmol) et le triphosgène (0,068 g, 0,23 mmol). Après 3h d'agitation à température ambiante, on hydrolyse et on extrait au dichlorométhane. La phase organique est séchée sur MgSO₄. Le brut est repris dans 10 mL de dichlorométhane et 8 mL de DMF anhydre. Après addition de 0,24 mL de triéthylamine, on ajoute le trans 4-amino-adamantan-1-ol (0,115 g, 0,69 mmol). Le milieu réactionnel est agité à 55°C pendant 16h. Après hydrolyse et extraction à l'acétate d'éthyle, la phase organique est lavée avec une solution aqueuse saturée en chlorure de sodium et séchée sur MgSO₄. Le brut obtenu est chromatographié sur gel de silice en éluant avec un gradient de méthanol dans le dichlorométhane variant de 0% à 10%. On obtient 0,09 g qui est trituré dans un mélange éthanol/acétate d'éthyle/diéthyléther pour conduire à 0,075 g d'endo-3-[4-(4-Methanesulfonyl-piperazin-1-yl)-phenoxy)-8-aza-bicyclo[3.2.1]octane-8-carboxylic acid (trans-5-hydroxy-adamantan-2-yl)-amide attendu.
Point de fusion = 252°C; [M+H⁺]= 559
**RMN 1 H (400 MHz, DMSO-d6) δ ppm** 6,9 (d, J = 8,9 Hz, 2H), 6,8 (d, J = 8,9 Hz, 2H), 5,8 (d, J = 6,3 Hz, 1H), 4,6 (tl, J = 4,3 Hz, 1H), 4,35 (s, 1H), 4,25 (sl, 2H), 3,7 (m, 1H), 3,25 (m, 4H), 3,1 (m, 4H), 2,9 (s, 3H), 2,1-1,55 (m, 19H), 1,3 (d, J = 2,6 Hz, 2H)

### EXEMPLE 5 :

### exo-3-[5-(4-Isopropoxy-phenyl)-pyridin-2-yloxy]-8-aza-bicyclo[3.2.1]octane-8-carboxylic acid (trans-5-carbamoyl-adamantan-2-yl)-amide (Composé n °9)

### 5.1/ endo-3-Hydroxy-8-aza-bicyclo[3.2.1]octane-8-carboxylic acid tert-butyl ester

On place la tropine (5 g, 35,4 mmol) dans 71 mL de dichloroéthane à 0°C sous azote. On ajoute le 1-chloroéthyl chloroformate (7,64 mL, 70,8 mmol). Le milieu réactionnel est agité 2h à 80)C. Après concentration à sec, on ajoute 40 mL de méthanol et on chauffe à 80°C pendant 1h15. Après évaporation du solvant, on dissout le brut dans 35 mL d'acétone et 35 mL d'eau. On ajoute NaHCO₃ (11,9 g, 141 mmol) et le t-butyldicarbonate (23,2 g, 106 mmol). Le milieu réactionnel est agité 16h à température ambiante. Après évaporation de l'acétone, le brut est hydrolysé et extrait à l'acétate d'éthyle. La phase organique est lavée avec une solution aqueuse saturée en chlorure de sodium et séchée sur MgSO₄. Le brut obtenu est trituré dans le pentane. L'insoluble obtenu est essoré et on obtient 6,05 g de endo-3-hydroxy-8-aza-bicyclo[3.2.1 ]octane-8-carboxylic acid tert-butyl ester attendu.
M^{+°} = 227

### 5.2/ exo-3-(4-Nitro-benzoyloxy)-8-aza-bicyclo[3.2.1]octane-8-carboxylic acid tert-butyl ester

On place l'endo-3-hydroxy-8-aza-bicyclo[3.2.1]octane-8-carboxylic acid tert-butyl ester (0,5 g, 2,2 mmol) dans 7,5 mL de THF anhydre en présence d'acide 4-nitrobenzoïque (0,39 g, 2,38 mmol) et de triphenylphosphine (0,692 g, 1,2 mmol). On ajoute lentement le DIAD (0,51 mL, 2,64 mmol). Le milieu réactionnel est agité à température ambiante pendant 20h. Après concentration à sec, le brut est chromatographié sur gel de silice en éluant avec un gradient d'acétate d'éthyle dans l'heptane variant de 0% à 30%. On obtient 0,66 g d'exo-3-(4-nitro-benzoyloxy)-8-aza-bicyclo[3.2.1]octane-8-carboxylic acid tert-butyl ester attendu.
[M+H⁺] = 377

### 5.3/ exo-3-Hydroxy-8-aza-bicyclo[3.2.1]octane-8-carboxylic acid tert-butyl ester

On place l'exo-3-(4-nitro-benzoyloxy)-8-aza-bicyclo[3.2.1]octane-8-carboxylic acid tert-butyl ester (0,66 g, 1,75 mmol) dans 6 mL de THF. On ajoute 0,9 mL de soude aqueuse 4N. Le milieu réactionnel est agité à température ambiante pendant 4h. On ajoute alors 15 mL d'eau et on extrait au dichlorométhane. La phase organique est lavée avec une solution saturée aqueuse de chlorure de sodium et séchée sur MgSO₄. Le brut obtenu est chromatographié sur gel de silice en éluant avec un gradient de méthanol dans le dichlorométhane variant de 0% à 10%. On obtient 0,32 g d'exo-3-hydroxy-8-aza-bicyclo[3.2.1]octane-8-carboxylic acid tert-butyl ester attendu.
M^{+°} = 227

### 5.4/ exo-3-(5-Bromo-pyridin-2-yloxy)-8-aza-bicyclo[3.2.1]octane-8-carboxylic acid tert-butyl ester

On place l'exo-3-hydroxy-8-aza-bicyclo[3.2.1]octane-8-carboxylic acid tert-butyl ester (0,2 g, 0,88 mmol) dans 1,5 mL de NMP. On ajoute la 5-bromo-2-pyridine (0,27 mL, 2,64 mmol) et le t-butylate de potassium (0,20 g, 1,76 mmol). Le milieu réactionnel est agité 10 min à température ambiante puis 15 min au micro-onde à 80°C. Après hydrolyse et extraction à l'acétate d'éthyle, la phase organique est lavée avec une solution aqueuse saturée en chlorure de sodium et séchée sur MgSO₄. Le brut obtenu est chromatographié sur gel de silice en éluant avec un gradient d'acétate d'éthyle dans l'heptane variant de 0% à 30%. On obtient 0,29 g d'exo-3-(5-bromo-pyridin-2-yloxy)-8-aza-bicyclo[3.2.1]octane-8-carboxylic acid tert-butyl ester attendu.
[M+H⁺] = 383

### 5.5/ exo- -3-[5-(4-Isopropoxy-phenyl)-pyridin-2-yloxy]-8-aza-bicyclo[3.2.1]octane-8-carboxylic acid tert-butyl ester

On place l'exo-3-(5-bromo-pyridin-2-yloxy)-8-aza-bicyclo[3.2.1]octane-8-carboxylic acid tert-butyl ester (0,5 g, 1,3 mmol) dans 13 mL de toluène en présence de tetrakistriphenylphosphine palladium (0,15 g, 0,13 mmol), de 4-isopropoxyphenyl boronic acid (0,235 g, 1,3 mmol) et 1,63 mL d'une solution 2M de carbonate de potassium. Le milieu réactionnel est agité au reflux pendant 16h. Après hydrolyse et extraction à l'acétate d'éthyle, la phase organique est lavée avec une solution aqueuse saturée en chlorure de sodium et séchée sur MgSO₄. Le brut obtenu est chromatographié sur gel de silice en éluant avec un gradient de méthanol dans le dichlorométhane variant de 0% à 10%. On obtient 0,33 g d'exo-3-[5-(4-isopropoxyphenyl)-pyridin-2-yloxy]-8-aza-bicyclo[3.2.1]octane-8-carboxylic acid tert-butyl ester attendu.
[M+H⁺] = 439

### 5.6/exo-3-[5-(4-isopropoxy-phenyl)-pyridin-2-yloxy]-8-aza-bicyclo[3.2.1]octane

Suivant un mode opératoire identique à celui décrit à l'étape 1.6, à partir d'exo-3-[5-(4-isopropoxy-phenyl)-pyridin-2-yloxy]-8-aza-bicyclo[3.2.1]octane-8-carboxylic acid tert-butyl ester (0,32 g, 0,73 mmol), le brut obtenu est concentré à sec, puis hydrolysé et extrait avec du diéthyléther. Puis la phase aqueuse est basifiée à pH 10 avec du K₂CO₃, et extraite au dichlorméthane. La phase organique est lavée avec une solution aqueuse saturée en chlorure de sodium, séchée sur MgSO₄ et concentrée à sec. On obtient 0,28 g d'exo-3-[5-(4-isopropoxy-phenyl)-pyridin-2-yloxy]-8-aza-bicyclo[3.2.1]octane attendu.
[M+H⁺] = 339

### 5.7/trans-4-Amino-adamantane-1-carboxylic acid

Le 4-oxo-adamantane-1-carboxylic acid (8 g, 41,2 mmol) est agité sous atmosphère d'H₂ en présence de palladium sur charbon 10% (0,5g) dans 160 mL d'une solution méthanolique d'ammoniac 7N pendant 19h. Le solide en suspension est tout d'abord essoré et lavé avec du méthanol, puis il est repris avec 200 mL d'eau et le palladium sur charbon est filtré. On concentre à sec, on reprend dans un peu de méthanol puis on essore le solide blanc qui est ensuite séché sur P₂O₅. On obtient 5,4 g de trans-4-amino-adamantane-1-carboxylic acid attendu.
[M+H⁺] = 196

### 5.8/ trans 4-tert-Butoxycarbonylamino-adamantane-1-carboxylic acid

On dissout le trans-4-amino-adamantane-1-carboxylic acid (7,74 g, 39,6 mmol) dans 70 mL d'une solution de soude aqueuse 1N puis on ajoute 70 mL de dioxane. On additionne le di-t-butoxycarbonate (25,9 g, 118,9 mmol). Le milieu réactionnel est agité 16h à température ambiante. Après évaporation du dioxane, la phase aqueuse est extraite au dichlorométhane. Puis on ajoute une solution aqueuse d'HCl 1N jusqu'à pH 4. On extrait au dichlorométhane. La phase organique est séchée sur MgSO₄ et concentrée à sec. On obtient 10,5 g de trans 4-t-butoxycarbonylamino-adamantane-1-carboxylic acid attendu.
[M+H⁺

### 5.9/ trans (5-Carbamoyl-adamantan-2-yl)-carbamic acid tert-butyl ester

On place le trans-4-t-butoxycarbonylamino-adamantane-1-carboxylic acid (5,8 g, 19,6 mmol) dans 100 mL de dichlorométhane sous azote. On ajoute la triéthylamine (54,7 mL, 393 mmol) à 0°C puis le chloroformiate d'éthyle (3,75 mL, 39,3 mmol). On maintient l'agitation à 0°C pendant 2h. On ajoute alors le chlorure d'ammonium (21 g, 393 mmol). Après 30 min à 0°C, l'agitation est poursuivie à température ambiante pendant 4h. Après hydrolyse, et extraction au dichlorométhane, la phase organique est séchée sur MgSO₄ et concentrée à sec. Le brut obtenu est chromatographié sur gel de silice en éluant avec un gradient d'acétate d'éthyle/méthanol dans l'heptane variant de 100/0/0 à 4/5/1. On obtient 4,0 g de trans-(5-carbamoyl-adamantan-2-yl)-carbamic acid tert-butyl ester.
[M+H⁺] = 295

### 5.10/ chlorhydrate du trans (5-Carbamoyl-adamantan-2-yl)-carbamic acid tert-butyl ester

On place le trans-(5-carbamoyl-adamantan-2-yl)-carbamic acid tert-butyl ester (4g, 13.59 mmol) dans 51 mL de HCl 4N dans le dioxane et on agite à température ambiante pendant 18h. On essore le précipité blanc obtenu et on lave au diéthyléther. On obtient 3,2 g du chlorhydrate de trans- 4-amino-adamantane-1-carboxylic acid amide.
[M+H⁺] = 195

### 5.11/ exo-3-[5-(4-Isopropoxy-phenyl)-pyridin-2-yloxy]-8-aza-bicyclo[3.2.1]octane-8-carboxylic acid (trans-5-carbamoyl-adamantan-2-yl)-amide

On place l'exo-3-[5-(4-isopropoxy-phenyl)-pyridin-2-yloxy]-8-aza-bicyclo[3.2.1]octane (0,28 g, 0,82 mmol) dans 8 mL de dichlorométhane à 0°C. Puis on ajoute la triéthylamine (0,23 mL, 1,64 mmol) et le triphosgène (0,097 g, 0,33 mmol). Après 3h d'agitation à température ambiante, on hydrolyse et on extrait au dichlorométhane. La phase organique est lavée avec une solution aqueuse saturée en chlorure de sodium et séchée sur MgSO₄. Le brut est repris dans 3 mL de DMF anhydre. On ajoute le chlorhydrate du trans-4-amino-adamantane-1-carboxylic acid amide (0,276 g, 1,2 mmol) solubilisé dans 5 mL de DMF en présence de diisopropyléthylamine (0,42 mL, 2,39 mmol). Le milieu réactionnel est agité à 50°C pendant 16h. Après hydrolyse et extraction au dichlorométhane, la phase organique est lavée avec une solution aqueuse saturée en chlorure de sodium et séchée sur MgSO₄. Le brut obtenu est chromatographié sur gel de silice en éluant avec un gradient de méthanol dans le dichlorométhane variant de 0% à 10%. On obtient 0,095 g d'exo-3-[5-(4-isopropoxy-phenyl)-pyridin-2-yloxy]-8-aza-bicyclo[3.2.1]octane-8-carboxylic acid (trans-5-carbamoyl-adamantan-2-yl)-amide attendu.
Point de fusion = 255°C; [M+H⁺] = 559
**RMN 1H (400 MHz, DMSO-d6) δ ppm :** 8,4 (d, J=2,5 Hz, 1H), 7,9 (dd, J=8,6 et 2,5 Hz, 1H), 7,55 (d, J=8,8 Hz, 2H), 7,0 (d, J=8,8 Hz, 2H), 6,95 (s, 1H), 6,8 (d, J=8,5 Hz, 1H), 7,0-6,7 (bs, 1H), 5,95 (d, J=6,2 Hz, 1H), 5,5 (m, 1H), 4,65 (m, 1H), 4,4 (m, 2H), 3,75 (m, 1 H), 2,1-1,3 (m, 27H).

### Exemple 6 :

### exo-3-[5-(4-Methoxy-phenyl)-pyrimidin-2-yloxy]-8-aza-bicyclo[3.2.1]octane-8-carboxylic acid (trans-5-carbamoyl-adamantan-2-yl)-amide-(Composé n°12)

### 6.1/ exo-3-[5-(4-Methoxy-phenyl)-pyrimidin-2-yloxy]-8-methyl-8-aza-bicyclo[3.2.1]octane

On place l'hydrure de sodium (0,25 g, 10,6 mmol) dans 15 mL de NMP anhydre sous azote et on ajoute lentement une solution de pseudo-tropanol (1 g, 7,1 mmol) solubilisé dans 5 mL de NMP anhydre. Le milieu réactionnel est agité 1h à 80°C. La solution est refroidie à température ambiante et on ajoute lentement la 2-methanesulfonyl-5-(**4**-methoxy-phenyl)-pyrimidine (2,81 g, 10,6 mmol) solubilisée dans 5 mL de NMP anhydre. Le milieu réactionnel est agité 1h30 à température ambiante puis 1h à 80°C. Après hydrolyse et extraction à l'acétate d'éthyle, la phase organique est lavée avec une solution aqueuse saturée en chlorure de sodium et séchée sur MgSO₄. Le brut obtenu est chromatographié sur gel de silice en éluant avec un gradient de méthanol/ammoniaque dans le dichlorométhane variant de 90/10/1 à 70/30/3 (dichlorométhane/méthanol/ammoniaque). On obtient 1,3 g d'exo-3-[5-(4-methoxy-phenyl)-pyrimidin-2-yloxy]-8-methyl-8-aza-bicyclo[3.2.1]octane attendu.
[M+H⁺] = 326

### 6.2/ exo-3-[5-(4-Methoxy-phenyl)-pyrimidin-2-yloxy]-8-aza-bicyclo[3.2.1]octane

On place l'exo-3-[5-(4-methoxy-phenyl)-pyrimidin-2-yloxy]-8-methyl-8-aza-bicyclo[3.2.1]octane (0,5 g, 1,54 mmol) dans 3 mL de dichloroéthane à 0°C sous azote. On ajoute le 1-chloroéthyl choroformate (0,33 mL, 3,1 mmol). Le milieu réactionnel est agité 18h à 80°C. Après concentration à sec, on ajoute 3 mL de méthanol et on chauffe à 80°C pendant 2h. Après évaporation du solvant, hydrolyse avec une solution aqueuse saturée en NaHCO₃ et extraction à l'acétate d'éthyle, la phase organique est séchée sur MgSO₄. Le brut obtenu est chromatographié sur gel de silice en éluant avec un gradient de méthanol/ammoniaque dans le dichlorométhane variant de 100/0/0 à 90/10/1 (dichlorométhane/méthanol/ammoniaque). On obtient 0,27 g d'exo-3-[5-(4-methoxy-phenyl)-pyrimidin-2-yloxy]-8-methyl-8-aza-bicyclo[3.2.1]octane attendu.
[M+H⁺] = 312

### 6.3/ exo-3-[5-(4-Methoxy-phenyl)-pyrimidin-2-yloxy]-8-aza-bicyclo[3.2.1]octane-8-carboxylic acid (trans-5-carbamoyl-adamantan-2-yl)-amide

On place l'exo-3-[5-(4-methoxy-phenyl)-pyrimidin-2-yloxy]-8-methyl-8-aza-bicyclo[3.2.1]octane (0,21 g, 0,66 mmol) dans 22 mL d'un mélange 50/50 de dichlorométhane/solution aqueuse saturée en NaHCO₃ à 0°C. On ajoute le phosgène 20% dans le toluène (0,52 mL, 0,99 mmol). Après 2h d'agitation, on extrait la phase aqueuse au dichlorométhane. La phase organique est séchée sur MgSO₄. Le brut obtenu est placé dans 7 mL de DMF anhydre sous azote. On ajoute la triéthylamine (0,23 mL, 1,65 mmol) suivie du chlorhydrate du trans 4-amino-adamantane-1-carboxylic acid amide (0,128 g, 0,66 mmol). Le milieu réactionnel est chauffé à 50°C pendant 16h. Après hydrolyse avec une solution aqueuse saturée en NaHCO₃ et extraction à l'acétate d'éthyle, la phase organique est lavée avec une solution aqueuse saturée en chlorure de sodium et séchée sur MgSO₄. Le brut obtenu est chromatographié sur gel de silice en éluant avec un gradient d'acétone/méthanol dans le dichlorométhane variant de 100/0/0 à 70/25/5 (dichlorométhane/acétone/méthanol). Après trituration dans un mélange acétate d'éthyle et diéthyléther, on obtient 0,138 g d'exo-3-[5-(4-Methoxy-phenyl)-pyrimidin-2-yloxy]-8-aza-bicyclo[3.2.1]octane-8-carboxylic acid (trans-5-carbamoyl-trans-adamantan-2-yl)-amide.
Point de fusion = 238 °C; [M+H⁺] = 532
**RMN 1H (400 MHz, DMSO-d6) δ ppm** 8,9 (s, 2H), 7,7 (d, J = 8,9 Hz, 2H), 7,1 (d, J = 8,8 Hz, 2H), 7,0 (bs, 1H), 6,7 (bs, 1H), 6,0 (d, J = 6,2 Hz, 1H), 5,45 (m, J = 5,5 Hz, 1H), 4,4 (m, 2H), 3,8 (s, 3H), 3,75 (m, 1 H), 2,1 (m, 2H), 2,05 - 1,65 (m, 17H), 1,4 (m, 2H)

### Exemple 7 :

### endo-3-Hydroxy-3-[4-(4-methanesulfonyl-piperazin-1-yl)-phenoxymethyl]-8-aza-bicyclo[3.2.1]octane-8-carboxylic acid (trans-5-hydroxy-adamantan-2-yl)-amide (Composé n°15)

### 7.1/ 8-benzyl-spiro[8-aza-bicyclo[3.2.1]octane-3,2'-oxirane]

On place le triméthyl sulfoxonium iodide (3,07 g, 13,9 mmol) dans 18 mL de DMSO sous azote en présence d'hydrure de sodium dispersé à 60% dans l'huile (0,56 g, 13,9 mmol). Après 30 min d'agitation à température ambiante, on ajoute lentement la 8-benzyl-8-azabicyclo[3.2.1]octan-3-one (2,0 g, 9,3 mmol) solubilisée dans 3 mL de DMSO. Le milieu réactionnel est maintenu 4h avec agitation puis 60h sans agitation. Après hydrolyse et extraction à l'acétate d'éthyle, la phase organique est lavée avec une solution aqueuse saturée en chlorure de sodium et séchée sur MgSO₄. On obtient 2,04 g de 8-benzyl-spiro[8-aza-bicyclo[3.2.1]octane-3,2'-oxirane] utilisé tel quel par la suite.
[M+H⁺] = 230

### 7.2/ Methanesulfonic acid 4-(4-methanesulfonyl-piperazin-1-yl)-phenyl ester

On place le 4-piperazin-1-yl-phenol (2,0 g, 11,2 mmol) dans 22 mL de pyridine à 0°C. On ajoute lentement le chlorure de mésyle (1,9 mL, 24,7 mmol). L'agitation est maintenue 2h à 0°C puis 2h à température ambiante. On ajoute ensuite 80 mL d'eau. Le précipité obtenu est essoré et lavé à l'eau, puis séché sur P₂O₅. On obtient 3,2 g de methanesulfonic acid 4-(4-methanesulfonyl-piperazin-1-yl)-phenyl ester attendu.
[M+H⁺] = 335

### 7.3/ 4-(4-Methanesulfonyl-piperazin-1-yl)-phenol

On place le methanesulfonic acid 4-(4-methanesulfonyl-piperazin-1-yl)-phenyl ester (3,2 g, 9,5 mmol) dans 29 mL d'une solution aqueuse de soude 1 N. Le milieu réactionnel est agité à reflux pendant 48h. On ajoute à température ambiante de l'acide acétique jusqu'à pH 4. Le précipité formé est essoré et lavé à l'eau puis séché sur P₂O₅. On obtient 2,6 g de 4-(4-methanesulfonyl-piperazin-1-yl)-phenol attendu.
[M+H⁺] = 257

### 7.4/ 8-Benzyl-3-[4-(4-methanesulfonyl-piperazin-1-yl)-phenoxymethyl]-8-aza-bicyclo[3.2.1]octan-3-endo-ol

On place le 4-(4-methanesulfonyl-piperazin-1-yl)-phenol (1,5 g, 5,7 mmol) dans 22 mL de DMF anhydre sous azote. On ajoute lentement l'hydrure de sodium dispersé à 60% dans l'huile (0,21 g, 5,3 mmol). Après 1h d'agitation à température ambiante, on ajoute le 8-benzyl-spiro[8-aza-bicyclo[3.2.1]octane-3,2'-oxirane] (1,1 g, 4,8 mmol) solubilisé dans 2 mL de DMF anhydre. Le milieu réactionnel est agité à 115°C pendant 5h. On rajoute alors du 8-benzyl-spiro[8-aza-bicydo[3.2.1]octane-3,2'-oxirane] (0,42 g, 1,6 mmol) et de l'hydrure de sodium dispersé à 60% dans l'huile (0,04 g, 1 mmol). L'agitation à 115°C est maintenue pendant 18h. Après hydrolyse avec une solution aqueuse saturée en NaHCO₃, et extraction à l'acétate d'éthyle et au dichlorométhane, la phase organique est lavée avec une solution aqueuse saturée en chlorure de sodium et séchée sur MgSO₄. On ajoute de l'acétate d'éthyle et l'insoluble formé est essoré. Le filtrat est concentré et chromatographié sur gel de silice en éluant avec un gradient de méthanol dans le dichlorométhane variant de 0% à 10%. On obtient 0,145 g de 8-benzyl-3-[4-(4-methanesulfonyl-piperazin-1-yl)-phenoxymethyl]-8-aza-bicyclo[3.2.1]octan-3-endo-ol attendu.
[M+H⁺] = 486

### 7.5/ 3-[4-(4-Methanesulfonyl-piperazin-1-yl)-phenoxymethyl]-8-aza-bicyclo[3.2.1]octan-3-endo-ol

On place le 8-benzyl-3-[4-(4-methanesulfonyl-piperazin-1-yl)-phenoxymethyl]-8-aza-bicyclo[3.2.1]octan-3-endo-ol (1,7 g, 3,0 mmol) dans 60 mL de méthanol. On ajoute le formiate d'ammonium (1,87 g, 29,7 mmol) et le palladium sur charbon 10%, ~50% dans l'eau (0,4 g). Le milieu réactionnel est chauffé au reflux pendant 1h30. Après filtration du catalyseur, le filtrat est concentré. Le brut obtenu est chromatographié sur gel de silice en éluant avec un gradient de méthanol/ammoniaque dans le dichlorométhane variant de 100/0/0 à 87/13/1,3 (dichlorométhane/méthanol/ammoniaque). On obtient 0,19 g de 3-[4-(4-methanesulfonyl-piperazin-1-yl)-phenoxymethyl]-8-aza-bicyclo[3.2.1]octan-3-endo-ol attendu.
[M+H⁺] = 396

### 7.6/ endo-3-Hydroxy-3-[4-(4-methanesulfonyl-piperazin-1-yl)-phenoxymethyl]-8-aza-bicyclo[3.2.1]octane-8-carboxylic acid (trans-5-hydroxy-adamantan-2-yl)-amide

On place le 3-[4-(4-methanesulfonyl-piperazin-1-yl)-phenoxymethyl]-8-aza-bicyclo[3.2.1]octan-3-endo-ol (0,6 g, 1,5 mmol) dans 51 mL d'un mélange 50/50 de dichlorométhane/solution aqueuse saturée en NaHCO₃ à 0 °C. On ajoute le phosgène 20% dans le toluène (1,2 mL, 2,28 mmol). Après 2h d'agitation, on rajoute du phosgène 20% dans le toluène (1 mL) et l'agitation est maintenue 30 min à 0°C. Après extraction de la phase aqueuse au dichlorométhane, la phase organique est séchée sur MgSO₄. Le brut obtenu est placé dans 7 mL de DMF anhydre sous azote. On ajoute la triéthylamine (0,21 mL, 1,48 mmol) suivie du trans-4-amino-adamantan-1-ol (0,14 g, 0,82 mmol) dissous dans 1,5 mL de DMF anhydre. Le milieu réactionnel est chauffé à 60°C pendant 16h. Après hydrolyse avec une solution aqueuse saturée en NaHCO₃, et extraction à l'acétate d'éthyle, la phase organique est lavée avec une solution aqueuse saturée en chlorure de sodium. Après concentration à sec, le brut obtenu est repris à l'acétonitrile et au méthanol à chaud. L'insoluble est filtré et le filtrat est chromatographié après concentration sur gel de silice en éluant avec un gradient de méthanol dans le dichlorométhane variant de 0% à 10%. On obtient 0,30 g d'endo-3-hydroxy-3-[4-(4-methanesulfonyl-piperazin-1-yl)-phenoxymethyl]-8-aza-bicyclo[3.2.1]octane-8-carboxylic acid (trans-5-hydroxy-adamantan-2-yl)-amide attendu.
Point de fusion = 249°C; [M+H⁺]= 589
**RMN 1 H (400 MHz, DMSO-d6) δ ppm :** 6,9 (d, J=9 Hz, 2H), 6,75 (d, J=9 Hz, 2H), 5,7 (d, J=6 Hz, 1 H), 4,5 (s, 1 H), 4,35 (s, 1H), 4,2 (m, 2H), 3,65 (m, 1 H), 3,4 (s, 2H), 3,2 (m , 4H), 3,1 (m, 4H), 2,9 (s, 3H), 2,2-1,2 (m, 21 H)

### Exemple 8 :

### endo-3-[4-(4-Methanesulfonyl-piperazin-1-yl)-phenoxymethyl]-8-aza-bicyclo[3.2.1]octane-8-carboxylic acid (trans-5-hydroxy-adamantan-2-yl)-amide

### (Composé n °17)

### 8.1/ 8-Aza-bicyclo[3.2.1]oct-2-ene-3,8-dicarboxylic acid 8-tert-butyl ester

On place le 3-trifluoromethanesulfonyloxy-8-aza-bicyclo[3.2.1]oct-2-ene-8-carboxylic acid tert-butyl ester (2,0 g, 5,6 mmol), l'hexacarbonylmolybdenum (0,74 g, 2,8 mmol), le diacétate de palladium (0,13 g, 0,56 mmol), le 1,1'-bis(diphenylphosphino)ferrocene (0,31, 0,56 mmol), la 4-diméthylaminopyridine (1,37 g, 11,2 mmol), la diisopropyléthylamine (2,24 mL, 12,9 mmol) dans 2,0 mL d'eau et 12 mL de dioxane dans un réacteur pour micro-onde. Le milieu est chauffé au micro-onde à 150°C pendant 10 min. Le brut est repris à l'eau et au dichlorométhane. La phase organique est extraite avec une solution aqueuse saturée en NaHCO₃. La phase aqueuse est acidifiée avec du KHSO₄ puis extraite à l'acétate d 'éthyle. La phase organique est lavée à l'eau puis avec une solution aqueuse saturée en chlorure de sodium et séchée sur MgSO₄. Après concentration à sec, on obtient 2,7 g de 8-aza-bicyclo[3.2.1]oct-2-ene-3,8-dicarboxylic acid 8-tert-butyl ester attendu.
[M+H⁺] = 253

### 8.2/ endo-8-Aza-bicyclo[3.2.1]octane-3,8-dicarboxylic acid 8-tert-butyl ester

On place 8-aza-bicyclo[3.2.1]oct-2-ene-3,8-dicarboxylic acid 8-tert-butyl ester (2,7 g, 10,7 mmol) dans 71 mL de méthanol. Puis on ajoute de l'oxyde de platine (0,24 g, 1,07 mmol) sous azote. Le milieu réactionnel est placé sous 50 psi d'hydrogène à 20°C. Après 6h d'agitation, le catalyseur est filtré et rincé au méthanol. Le filtrat est concentré à sec et chromatographié sur gel de silice en éluant avec un gradient d'acétate d'éthyle/méthanol dans l'heptane variant de 100/0/0 à 5/4,5/0,5 (heptane/acétate d'éthyle/méthanol). On obtient 1,12 g d'endo-8-aza-bicyclo[3.2.1]octane-3,8-dicarboxylic acid 8-tert-butyl ester attendu et 0,32 g d'exo-8-aza-bicyclo[3.2.1]octane-3,8-dicarboxylic acid 8-tert-butyl ester.
[M+H⁺] (-tBu) = 200

### 8.3/ endo-3-Hydroxymethyl-8-aza-bicyclo[3.2.1]octane-8-carboxylic acid tert-butyl ester

On place l'endo-8-aza-bicyclo[3.2.1]octane-3,8-dicarboxylic acid 8-tert-butyl ester (1,12 g, 4,4 mmol) dans 22 mL de THF anhydre sous azote à 0°C. On ajoute lentement le borane complexé par le THF, 1N dans le THF (17,5 mL, 17,5 mmol). A la fin de l'addition, le milieu réactionnel est agité à température ambiante pendant 1h30. L'hydrolyse est réalisée à 0°C en additionnant lentement de l'eau. On ajoute ensuite de l'acétate d'éthyle puis du KHSO₄. Après extraction à l'acétate d'éthyle, la phase organique est lavée avec une solution aqueuse saturée en NaHCO₃, puis à l'eau et enfin avec une solution aqueuse saturée en chlorure de sodium puis séchée sur MgSO₄. Après concentration à sec, on obtient 1,04 g de endo-3-hydroxymethyl-8-aza-bicyclo[3.2.1]octane-8-carboxylic acid tert-butyl ester attendu.
[M+H⁺] = 232

### 8.4/ endo-3-(4-Bromo-phenoxymethyl)-8-aza-bicyclo[3.2.1]octane-8-carboxylic acid tert-butyl ester

On place l'hydrure de sodium (0,16 g, 6,5 mmol) dans 5 mL de DMF anhydre sous azote. On ajoute lentement la tropine (1,04 g, 4,3 mmol) solubilisée dans 10 mL de DMF anhydre. Le milieu réactionnel est chauffé à 65°C pendant 1h puis on ajoute lentement le 4-fluorobenzène (1,13g, 6,5 mmol) placé dans 5 mL de DMF anhydre. Après agitation à 65°C pendant 5h, on rajoute de l'hydrure de sodium (0,05 g, 2,1 mmol) et le chauffage est maintenu à 65°C pendant 3h. Le milieu réactionnel est hydrolysé avec un mélange eau/glace. On extrait à l'acétate d'éthyle. La phase organique est lavée à l'eau puis avec une solution aqueuse saturée en chlorure de sodium et séchée sur MgSO₄. Le brut obtenu est chromatographié sur gel de silice en éluant avec un gradient d'acétate d'éthyle/méthanol dans l'heptane variant de 100/0/0 à 5/4,5/0,5 (heptane/acétate d'éthyle/méthanol). On obtient 1,05 g d'endo-3-(4-bromo-phenoxymethyl)-8-aza-bicyclo[3.2.1]octane-8-carboxylic acid tert-butyl ester attendu.
[M+H⁺] = 296

### 8.5/ endo-3-[4-(4-Methanesulfonyl-piperazin-1-yl)-phenoxymethyl]-8-aza-bicyclo[3.2.1]octane-8-carboxylic acid tert-butyl ester

On place d'endo-3-(4-bromo-phenoxymethyl)-8-aza-bicyclo[3.2.1]octane-8-carboxylic acid tert-butyl ester (1,05 g, 2,65 mmol), la 1-methanesulfonyl-piperazine (0,52 g, 3,2 mmol), le catalyseur 2'-(dimethylamino)-2-biphenylyl-palladium(II) chloride dinorbornylphosphine complex (0,22 g, 0,4 mmol), et le phosphate de potassium (1,41 g, 6,6 mmol) dans 13 mL de DME sous azote. Le milieu réactionnel est agité à 90°C pendant 4h30. Puis on ajoute la 1-methanesulfonyl-piperazine (0,13 g, 0,8 mmol), et le catalyseur 2'-(dimethylamino)-2-biphenylyl-palladium(II) chloride dinorbomylphosphine complex (0,075 g, 0,13 mmol). L'agitation à 90°C est maintenue pendant 16h. Après hydrolyse et extraction au dichlorométhane, la phase organique est lavée à l'eau puis avec une solution aqueuse saturée en chlorure de sodium et séchée sur MgSO₄. Le brut obtenu est chromatographié sur gel de silice en éluant avec un gradient de méthanol dans le dichlorométhane variant de 0% à 2,5%. On obtient 0,56 g d'endo-3-[4-(4-methanesulfonyl-piperazin-1-yl)-phenoxymethyl]-8-aza-bicyclo[3.2.1]octane-8-carboxylic acid tert-butyl ester attendu.
[M+H⁺] = 480

### 8.6/ endo-3-[4-(4-Methanesulfonyl-piperazin-1-yl)-phenoxymethyl]-8-aza-bicyclo[3.2.1]octane

On solubilise l'endo-3-[4-(4-methanesulfonyl-piperazin-1-yl)-phenoxymethyl]-8-aza-bicyclo[3.2.1]octane-8-carboxylic acid tert-butyl ester (0,56 g, 1,17 mmol) dans 2 mL de dichlorométhane. Puis on ajoute HCl 4N dans le dioxane (4,3 mL, 17,5 mmol). Après agitation 3h30 à température ambiante, le milieu réactionnel est concentré, repris à l'eau et avec une solution aqueuse de soude 1 N jusqu'à pH 10. Après extraction au dichlorométhane, lavage à l'eau puis avec une solution aqueuse saturée en chlorure de sodium, la phase organique est séchée sur MgSO₄ puis concentrée à sec. On obtient 0,42 g d'endo-3-[4-(4-methanesulfonyl-piperazin-1-yl)-phenoxymethyl]-8-aza-bicyclo[3.2.1]octane attendu utilisé tel quel par la suite.
[M+H⁺] = 380

### 8.7/ endo-3-[4-(4-Methanesulfonyl-piperazin-1-yl)-phenoxymethyl]-8-aza-bicyclo[3.2.1]octane-8-carboxylic acid (trans-5-hydroxy-adamantan-2-yl)-amide

On place l'endo-3-[4-(4-methanesulfonyl-piperazin-1-yl)-phenoxymethyl]-8-aza-bicyclo[3.2.1]octane (0,42 g, 1,1 mmol) dans 11 mL de dichlorométhane à 0°C sous azote. Puis on ajoute la triéthylamine (0,31 mL, 2,19 mmol) et le triphosgène (0,13 g, 0,44 mmol). Après 2h d'agitation à température ambiante, on hydrolyse et on extrait au dichlorométhane. La phase organique est lavée à l'eau puis avec une solution aqueuse saturée en chlorure de sodium et séchée sur MgSO₄. On obtient 0,48 g de brut. On place 0,24 g de ce brut dans 8 mL de DMF anhydre et on le coule sur une solution de trans-4-amino-adamantan-1-ol (0,104 g, 0,62 mmol) dans 1,5 mL de DMF et 0,11 mL de triéthylamine. Le milieu réactionnel est agité à 50°C pendant 16h. Après hydrolyse et extraction à l'acétate d'éthyle, la phase organique est lavée à l'eau puis avec une solution aqueuse saturée en chlorure de sodium et séchée sur MgSO₄. Après filtration à chaud dans un mélange méthanol/acétonitrile, les eaux-mères sont concentrées et le brut obtenu est chromatographié sur gel de silice en éluant avec un gradient de méthanol dans le dichlorométhane variant de 0% à 10%. On obtient 0,167 g de composé attendu qui est trituré dans de l'acétate d'éthyle puis essoré et séché pour conduire à 0,120 g d'endo-3-[4-(4-methanesulfonyl-piperazin-1-yl)-phenoxy]-8-aza-bicydo[3.2.1]octane-8-carboxylic acid (trans-5-hydroxy-adamantan-2-yl)-amide attendu.
Point de fusion = 227°C; [M+H⁺]= 573
**RMN 1 H (400 MHz, DMSO-d6) δ ppm** 6,9 (m, 4H), 5,75 (d, J = 6,3 Hz, 1 H), 4,35 (s, 1 H), 4,25 (m, 2H), 3,9 (d, J = 7,7 Hz, 2H), 3,65 (m, 1 H), 3,25 (m, 4H), 3,1 (m, 4H), 2,95 (s, 3H), 2,1 (m, 2H), 2,05 -1,8 (m, 8H), 1,7-1,55 (m, 8H), 1,4 (m, 2H), 1,3 (m, 2H)

### Exemple 9 :

### endo-3-[5-(4-tert-Butyl-piperazin-1-yl)-pyridin-2-yloxymethyl]-8-aza-bicyclo[3.2.1]octane-8-carboxylic acid (trans-5-hydroxy-adamantan-2-yl)-amide

### (Composé n°21)

### 9.1/ 1-tert-Butyl-4-(6-fluoro-pyridin-3-yl)-piperazine

On place la 1-tert-butylpiperazine (1,61 g, 11,4 mmol) dans 35 mL de toluène sous azote. Puis on ajoute la 5-bromo-2-fluoropyridine (2 g, 11,4 mmol), le t-butylate de sodium (1,6 g, 17 mmol), le trisdibenzylidene acetone dipalladium (0,52 g, 0,57 mmol), le S-PHOS (0,93 g, 17 mmol). Le milieu réactionnel est chauffé à 80°C pendant 18h. Après hydrolyse, et extraction à l'acétate d'éthyle, la phase organique est lavée à l'eau puis avec une solution aqueuse saturée en chlorure de sodium. Après séchage sur MgSO₄, et concentration à sec, le brut obtenu est chromatographié sur gel de silice en éluant avec un gradient de méthanol dans le dichlorométhane variant de 0% à 5%. On obtient 1,92 g de 1-tert-Butyl-4-(6-fluoro-pyridin-3-yl)-piperazine.
[M+H⁺] = 238

### 9.2/ endo-3-[4-(4-tert-Butyl-piperazin-1-yl)-phenoxymethyl]-8-aza-bicyclo[3.2.1]octane-8-carboxylic acid tert-butyl ester

On place l'hydrure de sodium (0,078 g, 3,11 mmol) dans 6,9 mL de DMF anhydre sous azote. On ajoute l'endo-3-Hydroxymethyl-8-aza-bicyclo[3.2.1]octane-8-carboxylic acid tert-butyl ester (0,5 g, 2, 07 mmol) dissous dans 9 mL de DMF anhydre à l'hydrure de sodium à 95% (0,078 g, 3,11 mmol) sous azote. Le milieu réactionnel est chauffé à 65°C pendant 1h. Puis on ajoute la 1-tert-Butyl-4-(6-fluoro-pyridin-3-yl)-piperazine (0,59 g, 2,5 mmol) dissoute dans 4 mL de DMF anhydre. Le milieu réactionnel est chauffé à 65°C pendant 1h30. On rajoute alors de l'hydrure de sodium à 95% (0,078 g, 3,11 mmol). Le milieu réactionnel est chauffé à 80°C pendant 1 h30 puis il est versé sur de la glace. Après extraction à l'acétate d'éthyle, la phase organique est lavée avec une solution aqueuse saturée en chlorure de sodium. Après séchage sur MgSO₄, et concentration à sec, le brut obtenu est chromatographié sur gel de silice en éluant avec un gradient de méthanol dans le dichlorométhane variant de 0% à 5%. On obtient 2,86 g d'un mélange qui est repris dans 25 mL de dichlorométhane, lavé à l'eau puis avec une solution aqueuse saturée en chlorure de sodium. Après séchage sur MgSO₄, et concentration à sec, on obtient 1,2 g d'endo-3-[4-(4-tert-Butyl-piperazin-1-yl)-phenoxymethyl]-8-aza-bicyclo[3.2.1]octane-8-carboxylic acid tert-butyl ester.
[M+H⁺] = 459

### 9.3/ endo-3-[4-(4-tert-Butyl-piperazin-1-yl)-phenoxymethyl]-8-aza-bicyclo[3.2.1]octane

On place l'endo-3-[4-(4-tert-Butyl-piperazin-1-yl)-phenoxymethyl]-8-aza-bicyclo[3.2.1]octane-8-carboxylic acid tert-butyl ester (1,2 g , 1,8 mmol) dans 3 mL de dichlorométhane. Le milieu réactionnel est refroidi à 0°C puis on ajoute lentement l'HCl 4N dans le dioxane (6,8 mL, 27,5 mmol). Après 4h d'agitation à température ambiante et concentration à sec, le brut obtenu est hydrolysé et lavé au diéthyl éther. Puis la solution aqueuse est basifiée avec du carbonate de potassium jusqu'à pH>10. Après extraction au dichlorométhane, la phase organique est lavée avec une solution aqueuse saturée en chlorure de sodium. Après séchage sur MgSO₄, et concentration à sec, le brut obtenu est chromatographié sur gel de silice en éluant avec un mélange méthanol/ammoniaque dans le dichlorométhane variant de 100/0/0 à 90/10/1 (dichlorométhane/méthanol/ammoniaque). On obtient 0,47 g d'endo-3-[4-(4-tert-Butyl-piperazin-1-yl)-phenoxymethyl]-8-aza-bicyclo[3.2.1]octane attendu.
[M+H⁺] = 359

### 9.4/ endo-3-[4-(4-tert-Butyl-piperazin-1-yl)-phenoxymethyl]-8-aza-bicyclo[3.2.1]octane-8-carboxylic acid (trans-5-hydroxy-adamantan-2-yl)-amide

On place l'endo-3-[4-(4-tert-Butyf-piperazin-1-yl)-phenoxymethyl]-8-aza-bicyclo[3.2.1]octane (0,72 g, 2 mmol) dans 20 mL de dichlorométhane sous azote. On ajoute la triéthylamine (0,56 mL, 4 mmol). Le milieu réactionnel est placé à 0°C et on ajoute le triphosgène (0,24 g, 0,8 mmol). Le mélange est agité à température ambiante pendant 2h. Après hydrolyse, et extraction au dichlorméthane, la phase organique est lavée à l'eau puis avec une solution aqueuse saturée en chlorure de sodium, séchée sur MgSO₄ et concentrée à sec. On obtient 0,97 g de brut. On place 0,28 g de ce brut dans 6 mL de DMF anhydre sous azote. On ajoute la triéthylamine (0,21 mL, 1,5 mmol) puis le chlorhydrate du trans-4-amino-adamantan-1-ol (0,145 g, 0,7 mmol). Le milieu réactionnel est agité à 50°C pendant 18h. Après filtration du milieu réactionnel, le filtrat est hydrolysé et extrait au dichlorométhane. Après lavage de la phase organique à l'eau puis avec une solution aqueuse saturée en chlorure de sodium, séchage sur MgSO₄ et concentration à sec, le brut obtenu est chromatographié sur gel de silice en éluant avec un mélange méthanol/ammoniaque dans le dichlorométhane variant de 100/0/0 à 95/5/0,5 (dichlorométhane/méthanol/ammoniaque). On obtient 0,04 g d'endo-3-[4-(4-tert-Butyl-piperazin-1-yl)-phenoxymethyl]-8-aza-bicyclo[3.2.1]octane-8-carboxylic acid (trans-5-hydroxy-adamantan-2-yl)-amide.
Point de fusion =211°C [M+H⁺] = 552
**1H NMR (400 MHz, DMSO-d6)** δ ppm 7,73 (d, J = 3,1 Hz, 1 H), 7,41 (dd, J = 9,1 Hz et 3 Hz, 1H), 6,69 (d, J = 9 Hz, 1H), 5,74 (d, J = 6,4 Hz, 1H), 4,33 (s, 1H), 4,27- 4,18 (m, 4H), 3,65 (m, 1 H), 3,02 (m, 4H), 2,64 (m, 4H), 2,15-1,81 (m, 9H), 1,71-1,55 (m, 7H), 1,38 (m, 2H), 1,28 (m, 2H), 1,05 (s, 9H)

### Exemple 10 :

### 3-[4-(4-Methanesulfonyl-piperazin-1-yl)-pyridyl]-3,8-diaza-bicyclo[3.2.1]octane-8-carboxylic acid (trans-5-hydroxy-adamantan-2-yl)-amide

### (Composé n°25)

### 10.1/ 3-(4-Bromo-pyridyl)-3,8-diaza-bicyclo[3.2.1]octane-8-carboxylic acid tert-butyl ester

On place le 3,8-diaza-bicyclo[3.2.1]octane-8-carboxylic acid tert-butyl ester (0,4 g, 1,9 mmol), la 2,5-dibromopyridine (0,58 g, 2,45 mmol), le trisdibenzylidene acetone dipalladium (0,07 g, 0,08 mmol), le Xantphos (0,131 g, 0,23 mmol) et le t-butylate de sodium (0,27 g, 2,8 mmol) dans 19 mL de toluène sous azote. Le milieu réactionnel est agité au reflux pendant 4h. Après hydrolyse et extraction à l'acétate d'éthyle, la phase organique est lavée avec une solution aqueuse saturée en chlorure de sodium et séchée sur MgSO₄. Le brut obtenu est chromatographié sur gel de silice en éluant avec un gradient d'acétate d'éthyle dans l'heptane variant de 0% à 30%. On obtient 0,54 g de 3-(4-bromo-pyridyl)-3,8-diaza-bicyclo[3.2.1]octane-8-carboxylic acid tert-butyl ester attendu.
[M+H⁺] = 368

### 10.2/ 3-[4-(4-Methanesulfonyl-piperazin-1-yl)-pyridyl]-3,8-diaza-bicyclo[3.2.1]octane-8-carboxylic acid tert-butyl ester

On place le 3-(4-bromo-phenyl)-3,8-diaza-bicyclo[3.2.1]octane-8-carboxylic acid tert-butyl ester (0,64 g, 1,7 mmol)), la 1-methanesulfonyl-piperazine (0,43 g, 2,6 mmol), le trisdibenzylidene acetone dipalladium (0,069 g, 0,07 mmol), le S-Phos (0,11 g, 0,28 mmol), et le t-butylate de sodium (0,25 g, 2,6 mmol) dans 10 mL de toluène sous azote. Le milieu réactionnel est agité au reflux pendant 1h30. Après hydrolyse avec une solution aqueuse saturée en NaHCO₃, et extraction à l'acétate d'éthyle, la phase organique est lavée avec une solution aqueuse saturée en chlorure de sodium et séchée sur MgSO₄. Le brut obtenu est chromatographié sur gel de silice en éluant avec un gradient de méthanol/acétate d'éthyle dans le dichlorométhane variant de 100/0/0 à 7/2,5/0,5 (dichlorométhane/acétate d'éthyle/méthanol). On obtient 0,75 g de 3-[4-(4-methanesulfonyl-piperazin-1-yl)-phenyl]-3,8-diaza-bicyclo[3.2.1]octane-8-carboxylic acid tert-butyl ester attendu.
[M+H⁺] = 452

### 10.3/ 3-[4-(4-Methanesulfonyl-piperazin-1-yl)-phenyl]-3,8-diaza-bicyclo[3.2.1]octane

On solubilise le 3-[4-(4-methanesulfonyl-piperazin-1-yl)-pyridyl]-3,8-diaza-bicyclo[3.2.1]octane-8-carboxylic acid tert-butyl ester (0,75 g, 1,66 mmol) dans 16 mL de dioxane. Puis on ajoute HCl 4N dans le dioxane (4,3 mL, 17,5 mmol) suivi d'environ 3 mL de méthanol. Après agitation 16h à température ambiante, le milieu réactionnel est concentré, repris à l'eau. Après extraction au diéthyléther, le pH de la phase aqueuse est amené à pH10 avec K₂CO₃ en présence de dichlorométhane. Après extraction au dichlorométhane, la phase organique est lavée avec une solution aqueuse saturée en chlorure de sodium, et séchée sur MgSO₄ puis concentrée à sec. On obtient 0,59 g de 3-[4-(4-methanesulfonyl-piperazin-1-yl)-pyridyl]-3,8-diaza-bicyclo[3.2.1]octane attendu utilisé tel quel par la suite.
[M+H⁺] = 352

### 10.4/ 3-[4-(4-Methanesulfonyl-piperazin-1-yl)-pyridyl]-3,8-diaza-bicyclo[3.2.1]octane-8-carboxylic acid (trans-5-hydroxy-adamantan-2-yl)-amide

On place le 3-[4-(4-methanesulfonyl-piperazin-1-yl)-pyridyl]-3,8-diaza-bicyclo[3.2.1]octane (0,46 g, 1,32 mmol) dans 13 mL de dichlorométhane à 0°C sous azote. Puis on ajoute la triéthylamine (0,37 mL, 2,64 mmol) et le triphosgène (0,157 g, 0,53 mmol). Après 3h d'agitation à température ambiante, on hydrolyse avec un mélange eau/glace et on extrait au dichlorométhane. La phase organique est lavée avec une solution aqueuse saturée en chlorure de sodium et séchée sur MgSO₄. On obtient 0,57 g de brut. On place 0,20 g de ce brut dans 5 mL de DMF anhydre. On ajoute la triéthylamine (0,17 mL, 1,21 mmol) puis le chlorhydrate du trans-4-amino-adamantan-1-ol (0,12 g, 0,58 mmol). Le milieu réactionnel est agité à 50°C pendant 16h. Après hydrolyse et extraction au dichlorométhane, la phase organique est lavée à l'eau puis avec une solution aqueuse saturée en chlorure de sodium et séchée sur MgSO₄. Le brut obtenu est chromatographié sur gel de silice en éluant avec un gradient de méthanol dans le dichlorométhane variant de 0% à 10%. On obtient 0,13 g de composé attendu qui est trituré dans le diisopropyléther puis essoré et séché pour conduire à 0,84 g de 3-[4-(4-methanesulfonyl-piperazin-1-yl)-pyridyl]-3,8-diaza-bicyclo[3.2.1]octane-8-carboxylic acid (trans-5-hydroxy-adamantan-2-yl)-amide attendu.
Point de fusion = 243°C; [M+H⁺] = 545
**RMN 1 H (400 MHz, DMSO-d6) δ ppm :** 7,9 (d, J=2,8 Hz, 1 H) ; 7,5 (dd, J=9,2 et 2,8 Hz, 1H), 6,7 (d, J=9,2 Hz, 1 H), 6,1 (d, J=6,2 Hz, 1H), 4,4 (s, 2H), 4,3 (s,1H), 3,85 (d, J=11,7 Hz,2H), 3,7 (m, 1H), 3,2 (m, 4H), 3,1 (m, 4H), 2,95 (s, 3H), 2,85 (m, 2H), 2,0-1,3 (m, 17H)

Le tableau qui suit illustre les structures chimiques et les propriétés physiques de quelques exemples de composés selon l'invention. Dans ce tableau:
- Me, Et, n-Pr, i-Pr, n-Bu et i-Bu représentent respectivement des groupes méthyle, éthyle, n-propyle, isopropyle, n-butyle et isobutyle, et
- Ph et Bn représentent respectivement des groupes phényle et benzyle.
- la colonne PF indique le point de fusion, en °C, du composé, et
- Les spectres de résonnance magnétique du proton (RMN¹H), tels que décrits ci-dessous, sont enregistrés à 400MHz dans du DMSO-d6, en utilisant le pic du DMSO-d5 comme référence. Les déplacements chimiques δ sont exprimés en partie par million (ppm). Les signaux observés sont exprimés ainsi : s = singulet ; d = doublet ; t = triplet ; m = massif ou singulet large ; H = proton (pour les rotamères, on note H*_{M}* et H*ₘ* en référence aux isomères majoritaires ou minoritaires *M* et *m* respectivement).
- dans la colonne CUSM, sont indiqués successivement, la méthode analytique de chromatographie liquide haute performance utilisée (A, B ou C) et détaillée ci-dessous, le temps de rétention du composé exprimé en minute, et le pic MH⁺ identifié par spectrométrie de masse.
   - Méthode A : UPLC/TOF
      Colonne : Acquity BEH C18, 50x2,1 mm, 1,7 µm
      Solvant A : H₂O + 0,05% TFA; solvant B : ACN + 0,035%TFA ; débit = 1 mL/min
      Gradient 3 min: T0 **:** 98% A - T1,6 à T2,1 min : 100%B - T2,5 à T3min : 98%A
      T°=40°C
      Détection : 220 nM
      Ionisation: ESI+
   - Méthode B : HPLC/ZQ CH3COONH4 5mM /ACN
      Colonne : Kromasil C18, 50x2,1 mm, 3,5µm
      Solvant A : CH3COONH4 + 3% ACN; solvant B : ACN; débit = 0,8 mL/min
      Gradient 10 min :T0:100% A - T5,5 à T7 min: 100% B - T7,1 à T10 min : 100% A
      T°= 40°C
      Détection : 220 nM
      Ionisation: ESI+

**Tableau**

| | | | | | | | | | | | |
|---|---|---|---|---|---|---|---|---|---|---|---|
| 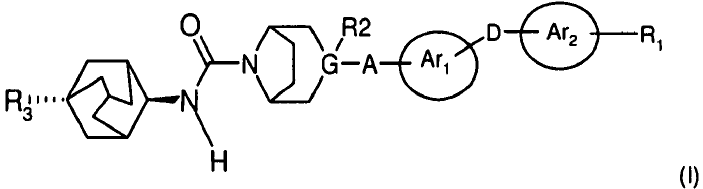 | | | | | | | | | | | |
| | La voie A correspond aux Schémas 1 et 2 | | | | | | | | | | |
| | La voie B correspond aux Schémas 1,3a et 4 | | | | | | | | | | |
| | La voie Ca correspond aux Schémas Schéma 1, 3a et 5 | | | | | | | | | | |
| | La voie Cb correspond aux Schémas Schéma 1,3b et 5 | | | | | | | | | | |
| | La voie D correspond aux Schémas Schéma 1 et 7 | | | | | | | | | | |
| | La voie E correspond aux Schémas Schéma 1 et 8 | | | | | | | | | | |
| | La voie F correspond aux Schémas Schéma 1 et 6 | | | | | | | | | | |
| | Dans le tableau, les flèches indiquent les positions des liaisons avec l'atome adajacent A ou D | | | | | | | | | | |
| | * le signe « - » signifie une liaison | | | | | | | | | | |
| | ** le signe « # » signifie que R2 est absent | | | | | | | | | | |

| **N°** | **G** | A* | **D*** | **Ar1** | **Ar2** | **R1** | **R2**** | **R3** | **PF** | **LC-MS** | **Voie** |
|---|---|---|---|---|---|---|---|---|---|---|---|
| 1 | C | - | - | 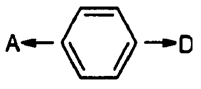 | 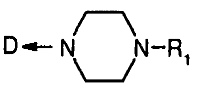 | -SO₂-Me | H | OH | 250 | A 1,79 543 | A |
| 2 | C | - | O | 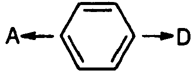 | 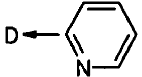 | H | H | OH | 103 | A | A |
| | | | | | | | | | | 1,08 | |
| | | | | | | | | | | 474 | |
| 3 | C | - | - | 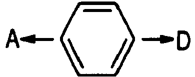 | 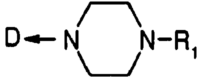 | -SO₂-Me | OH endo | OH | >250 | B | B |
| | | | | | | | | | | 3,15 | |
| | | | | | | | | | | 559 | |
| 4 | C | | - | 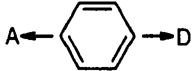 | 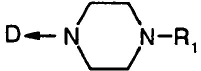 | -SO₂-Me | OH endo | -C(O)NH₂ | 276 | B | B |
| | | | | | | | | | | 3,1 | |
| | | | | | | | | | | 586 | |
| 5 | C | O | - | 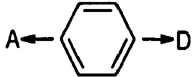 | 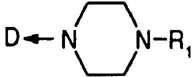 | -SO₂-Me | H endo | OH | 146 | A | Ca |
| | | | | | | | | | | 0,86 | |
| | | | | | | | | | | 559 | |
| 6 | C | O | - | 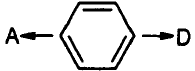 | 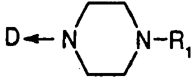 | -SO₂-Me | H endo | -C(O)NH₂ | 247 | A | Ca |
| | | | | | | | | | | 0,83 | |
| | | | | | | | | | | 586 | |
| 7 | C | O | - | 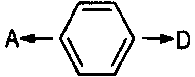 | 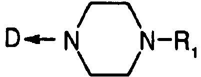 | -SO₂-Me | H exo | OH | 252 | A | Ca |
| | | | | | | | | | | 0,89 | |
| | | | | | | | | | | 559 | |
| 8 | C | O | - | 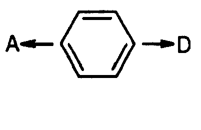 | 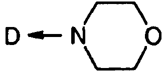 | H | H endo | -C(O)NH₂ | 220 | A | Ca |
| | | | | | | | | | | 0,74 | |
| | | | | | | | | | | 509 | |
| 9 | C | O | - | 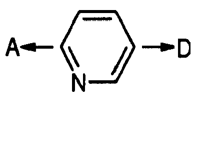 | 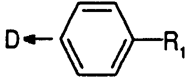 | -OiPr | H endo | -C(O)NH₂ | 255 | A | Cb |
| | | | | | | | | | | 1,25 | |
| | | | | | | | | | | 559 | |
| 10 | C | O | - | 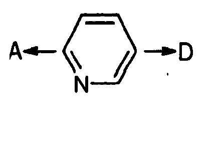 | 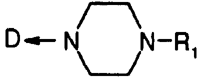 | -SO₂-Me | H endo | -C(O)NH₂ | 270 | B | D |
| | | | | | | | | | | 3,33 | |
| | | | | | | | | | | 587 | |
| 11 | C | O | - | 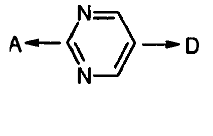 | 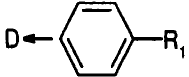 | OMe | H endo | OH | 196 | A | D |
| | | | | | | | | | | 1,09 | |
| | | | | | | | | | | 505 | |
| 12 | C | O | - | 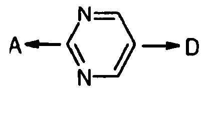 | 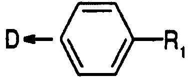 | OMe | H endo | -C(O)NH₂ | 238 | A | D |
| | | | | | | | | | | 1,06 | |
| | | | | | | | | | | 532 | |
| 13 | C | -CH₂-O- | - | 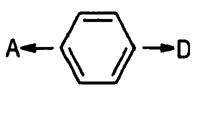 | 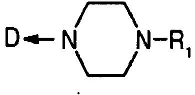 | -SO₂-Me | H endo | OH | 225 | A | Ca |
| | | | | | | | | | | 0,92 | |
| | | | | | | | | | | 573 | |
| 14 | C | -CH₂-O- | - | 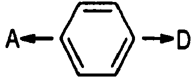 | 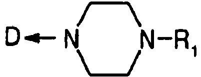 | -SO₂-Me | H endo | -C(O)NH₂ | 253 | A | Ca |
| | | | | | | | | | | 0,9 | |
| | | | | | | | | | | 600 | |
| 15 | C | -CH₂-O- | - | 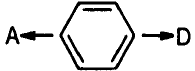 | 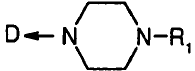 | -SO₂-Me | OH endo | OH | 249 | A | E |
| | | | | | | | | | | 0,77 | |
| | | | | | | | | | | 589 | |
| 16 | C | -CH₂-O- | - | 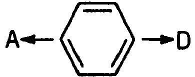 | 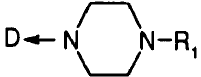 | -SO₂-Me | OH endo | -C(O)NH₂ | 210 | A | E |
| | | | | | | | | | | 0,75 | |
| | | | | | | | | | | 616 | |
| 17 | C | -CH₂-O- | - | 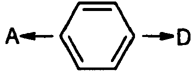 | 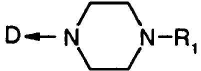 | -SO₂-Me | H exo | OH | 227 | A | Ca |
| | | | | | | | | | | 0,91 | |
| | | | | | | | | | | 573 | |
| 18 | C | -CH₂-O- | - | 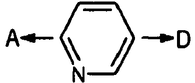 | 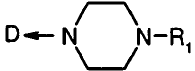 | -SO₂-Me | H exo | OH | 230 | A | D |
| | | | | | | | | | | 0,85 | |
| | | | | | | | | | | 574 | |
| 19 | C | -CH₂-O- | - | 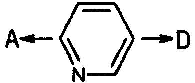 | 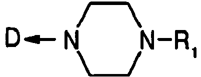 | -SO₂-Me | H exo | -C(O)NH₂ | 228 | A | D |
| | | | | | | | | | | 0,82 | |
| | | | | | | | | | | 601 | |
| 20 | C | -CH₂-O- | - | 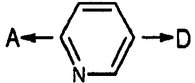 | 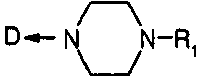 | -t-butyl | H exo | -C(O)NH₂ | 220 | A | D |
| | | | | | | | | | | 0,73 | |
| | | | | | | | | | | 579 | |
| 21 | C | -CH₂-O- | - | 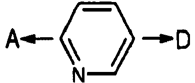 | 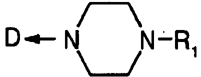 | -t-butyl | H exo | OH | 211 | A | D |
| | | | | | | | | | | 0,74 | |
| | | | | | | | | | | 552 | |
| 22 | C | -CH₂-O- | - | 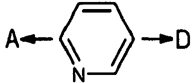 | 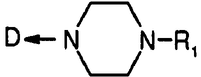 | -SO₂-Me | H endo | -C(O)NH₂ | 250 | A | D |
| | | | | | | | | | | 0,84 | |
| | | | | | | | | | | 601 | |
| 23 | C | -CH₂-O- | - | 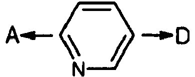 | 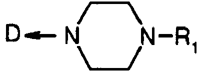 | -t-buty | H endo | OH | 273 | A | D |
| | | | | | | | | | | 0,75 | |
| | | | | | | | | | | 552 | |
| 24 | C | -CH₂-O- | - | 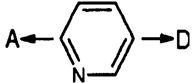 | 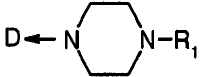 | -t-butyl | H endo | -C(O)NH₂ | 260 | A | D |
| | | | | | | | | | | 0,74 | |
| | | | | | | | | | | 579 | |
| 25 | N | - | - | 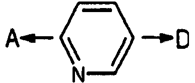 | 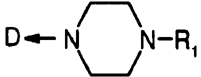 | -SO₂-Me | # | OH | 243 | A | F |
| | | | | | | | | | | 0,66 | |
| | | | | | | | | | | 545 | |
| 26 | N | - | - | 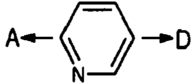 | 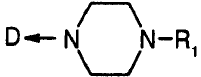 | -SO₂-Me | # | -C(O)NH₂ | >250 | A | F |
| | | | | | | | | | | 0,65 | |
| | | | | | | | | | | 572 | |
| 27 | C | O | - | 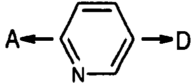 | 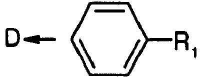 | -Si(Me)₃ | H endo | -C(O)NH₂ | 276 | A | Cb |
| | | | | | | | | | | 1,57 | |
| | | | | | | | | | | 573 | |

Les composés selon l'invention ont fait l'objet d'essais pharmacologiques permettant de déterminer leur effet inhibiteur de l'enzyme 11beta-HSD1 qui est une enzyme qui intervient dans le métabolisme des lipides ou le métabolisme du glucose.

Ces essais ont consisté à mesurer l'activité inhibitrice *in vitro* des composés de l'invention grâce à un test SPA (Scintillation Proximity Assay) en format 384 puits. La protéine 11 bêta-HSD1 recombinante a été produite en levure *S.cerevisiae.* La réaction a été réalisée en incubant l'enzyme en présence de ³H-cortisone et de NADPH, en absence ou en présence de concentration croissante d'inhibiteur. Des billes SPA couplées à un anticorps anti-souris, préincubées avec un anticorps anti-cortisol, ont permis de mesurer la quantité de cortisol formé au cours de la réaction.

L'activité inhibitrice vis-à-vis de l'enzyme 11bêta HSD1 est donnée par la concentration qui inhibe 50% de l'activité de 11bêta-HSD1 (Cl₅₀).

Les Cl₅₀ des composés selon l'invention sont inférieures à 1 µM. Par exemple, les Cl₅₀ des composés n ° 5 et 17 sont respectivement de 0,010 et 0,004 µM.

Il apparaît donc que les composés selon l'invention ont une activité inhibitrice de l'enzyme 11béta-HSD1. Les composés selon l'invention peuvent donc être utilisés pour la préparation de médicaments, en particulier de médicaments inhibiteurs de l'enzyme 11béta-HSD1.

Ainsi, selon un autre de ses aspects, l'invention a pour objet des médicaments qui comprennent un composé de formule (I), ou un sel d'addition de ce dernier à un acide pharmaceutiquement acceptable, ou encore un solvat du composé de formule (I).

Ces médicaments trouvent leur emploi en thérapeutique, notamment dans le traitement de l'obésité, des diabètes, de la résistance à l'insuline, du syndrome métabolique, du syndrome de Cushing, de l'hypertension, de l'athérosctérose, de la cognition et de la démence, des glaucomes, de l'ostéoporose et de certaines maladies infectieuses en augmentant l'efficacité du système immunitaire.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention ou un sel pharmaceutiquement acceptable, un hydrate ou solvat dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

La présente invention, selon un autre de ses aspects, décrit également une méthode de traitement des pathologies ci-dessus indiquées qui comprend l'administration, à un patient, d'une dose efficace d'un composé selon l'invention, ou un de ses sels pharmaceutiquement acceptables ou solvats.

## Revendications

1. Composé répondant à la formule (I) 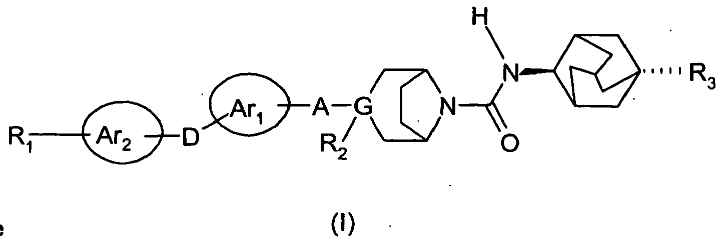 dans laquelle
A représente une liaison ou un groupe -O-(CH₂)ₙ- avec n est un nombre égal à 0 ou 1,
D représente une liaison ou un atome d'oxygène,
G représente un atome de carbone ou d'azote,
Ar1 représente un groupe aryle ou hétéroaryle,
Ar2 représente un groupe aryle ou hétéroaryle ou hétérocycloalkyle,
R1 représente un atome d'hydrogène, un groupe (C1-C6)alkyle, -SO₂-(C1-C6)alkyle, -SO₂-halogéno(C1-C6)alkyle, (C1-C6)alcoxy ou -Si(alkyle)₃,
R2 représente un atome d'hydrogène ou un groupe hydroxy ou, quand G est un atome d'azote, R2 est absent,
R3 représente un groupe hydroxy ou -C(O)-NH₂,
à l'état de base ou de sel d'addition à un acide.

2. Composé de formule (I) selon la revendication 1, **caractérisé en ce que** les substituants du groupe Ar1 sont situés en position para l'un par rapport à l'autre.

3. Composé de formule (I) selon la revendication 1, **caractérisé en ce que** les substituants du groupe Ar2 sont situés en position para l'un par rapport à l'autre.

4. Composé de formule (I) selon la revendication 1, **caractérisé en ce que** G représente un atome de carbone.

5. Composé de formule (I) selon la revendication 1, **caractérisé en ce que** G représente un atome d'azote.

6. Composé de formule (I) selon la revendication 1, **caractérisé en ce que** R3 représente -C(O)-NH₂.

7. Procédé de préparation d'un composé de formule (I) selon l'une quelconque des revendications 1 à 6, **caractérisé en ce que** l'on fait réagir un composé de formule (IV): 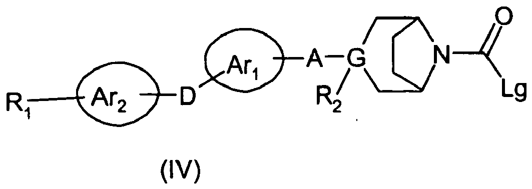 dans laquelle A, D, G, Ar1, Ar2, R1, R2 sont tels que définis dans la revendication 1 et Lg est un groupe partant
avec un composé de formule (V): 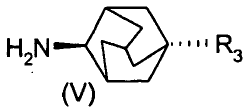 dans laquelle R3 est tel que défini dans la revendication 1
en présence d'un solvant et d'une base.

8. Médicament, **caractérisé en ce qu'**il comprend un composé de formule (I) selon l'une quelconque des revendications 1 à 6, ou un sel d'addition de ce composé à un acide pharmaceutiquement acceptable.

9. Composition pharmaceutique, **caractérisée en ce qu'**elle comprend un composé de formule (I) selon l'une quelconque des revendications 1 à 6, ou un sel pharmaceutiquement acceptable, ainsi qu'au moins un excipient pharmaceutiquement acceptable.

10. Composé de formule (I) selon l'une quelconque des revendications 1 à 6 pour son utilisation dans le traitement de l'obésité, des diabètes, de la résistance à l'insuline, du syndrome métabolique, du syndrome de Cushing, de l'hypertension, de l'athérosclérose, de la cognition et de la démence, des glaucomes, de l'ostéoporose et de certaines maladies infectieuses en augmentant l'efficacité du système immunitaire.

## Patentansprüche

1. Verbindung der Formel (I) 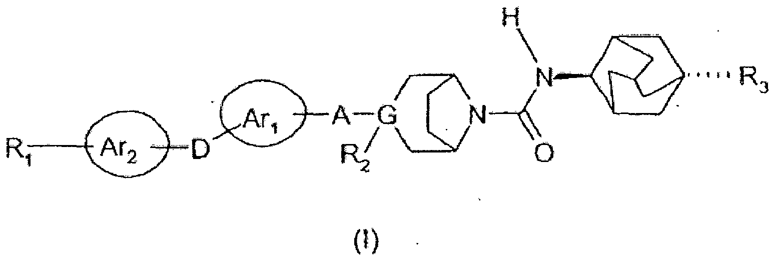 worin
A für eine Bindung oder eine -O-(CH₂)ₙ-Gruppe, wobei n eine Zahl mit einem Wert von 0 oder 1 bedeutet, steht,
D für eine Bindung oder ein Sauerstoffatom steht,
G für ein Kohlenstoff- oder Stickstoffatom steht,
Ar1 für eine Aryl- oder Heteroarylgruppe steht,
Ar2 für eine Aryl- oder Heteroaryl- oder Heterocycloalkylgruppe steht,
R1 für ein Wasserstoffatom oder eine (C1-C6)Alkyl-, -SO₂-(C1-C6)Alkyl-, -SO₂-Halogen(C1-C6)alkyl-, (C1-C6)Alkoxy- oder -Si(Alkyl)₃-Gruppe steht,
R2 für ein Wasserstoffatom oder eine Hydroxygruppe steht oder dann, wenn G für ein Stickstoffatom steht, fehlt,
R3 für eine Hydroxy- oder -C(O)-NH₂-Gruppe steht,
in Basen- oder Säureadditionssalzform.

2. Verbindung der Formel (I) nach Anspruch 1, **dadurch gekennzeichnet, dass** die Substituenten der Gruppe Ar1 in para-Position zueinander stehen.

3. Verbindung der Formel (I) nach Anspruch 1, **dadurch gekennzeichnet, dass** die Substituenten der Gruppe Ar2 in para-Position zueinander stehen.

4. Verbindung der Formel (I) nach Anspruch 1, **dadurch gekennzeichnet, dass** G für ein Kohlenstoffatom steht.

5. Verbindung der Formel (I) nach Anspruch 1, **dadurch gekennzeichnet, dass** G für ein Stickstoffatom steht.

6. Verbindung der Formel (I) nach Anspruch 1, **dadurch gekennzeichnet, dass** R3 für -C(O)-NH₂ steht.

7. Verfahren zur Herstellung einer Verbindung der Formel (I) gemäß einem der Ansprüche 1 bis 6, **dadurch gekennzeichnet, dass** man eine Verbindung der Formel (IV): 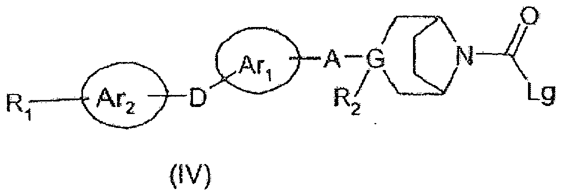 worin A, D, G, Ar1, Ar2, R1 und R2 wie in Anspruch 1 definiert sind und Lg für eine Abgangsgruppe steht,
in Gegenwart eines Lösungsmittels und einer Base mit einer Verbindung der Formel (V): 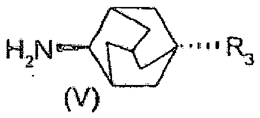 worin R3 wie in Anspruch 1 definiert ist,
umsetzt.

8. Arzneimittel, **dadurch gekennzeichnet, dass** es eine Verbindung der Formel (I) gemäß einem der Ansprüche 1 bis 6 oder ein Additionssalz dieser Verbindung mit einer pharmazeutisch unbedenklichen Säure umfasst.

9. Pharmazeutische Zusammensetzung, **dadurch gekennzeichnet, dass** sie eine Verbindung der Formel (I) gemäß einem der Ansprüche 1 bis 6 oder ein pharmazeutisch unbedenkliches Salz sowie mindestens einen pharmazeutisch unbedenklichen Hilfsstoff umfasst.

10. Verbindung der Formel (I) nach einem der Ansprüche 1 bis 6 zur Verwendung bei der Behandlung von Obesitas, Diabetes, Insulinresistenz, metabolischem Syndrom, Cushing-Syndrom, Hypertonie, Atherosklerose, Kognition und Demenz, Glaukomen, Osteoporose und bestimmten Infektionskrankheiten durch Erhöhung der Effizienz des Immunsystems.

## Claims

1. A compound corresponding to formula (I) 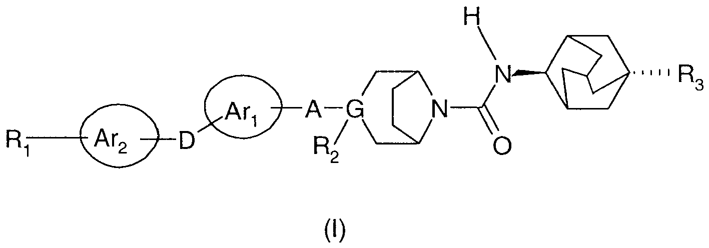 in which
A is a bond or an -O-(CH₂)ₙ- group with n being a number equal to 0 or 1,
D is a bond or an oxygen atom,
G is a carbon or nitrogen atom,
Ar1 is an aryl or heteroaryl group,
Ar2 is an aryl or heteroaryl or heterocycloalkyl group,
R1 is a hydrogen atom, or a (C1-C6)alkyl, -SO₂-(C1-C6)alkyl, -SO₂-halo(C1-C6)alkyl, (C1-C6)alkoxy or -Si(alkyl)₃ group,
R2 is a hydrogen atom or a hydroxyl group or, when G is a nitrogen atom, R2 is absent,
R3 is a hydroxyl or -C(O)-NH₂ group,
in the form of a base or of an addition salt with an acid.

2. A compound of formula (I) as claimed in claim 1, **characterized in that** the substituents of the Ar1 group are in the para-position with respect to one another.

3. A compound of formula (I) as claimed in claim 1, **characterized in that** the substituents of the Ar2 group are in the para-position with respect to one another.

4. A compound of formula (I) as claimed in claim 1, **characterized in that** G is a carbon atom.

5. A compound of formula (I) as claimed in claim 1, **characterized in that** G is a nitrogen atom.

6. A compound of formula (I) as claimed in claim 1, **characterized in that** R3 is -C(O)-NH₂.

7. A process for preparing a compound of formula (I) as claimed in any one of claims 1 to 6, **characterized in that** a compound of formula (IV): 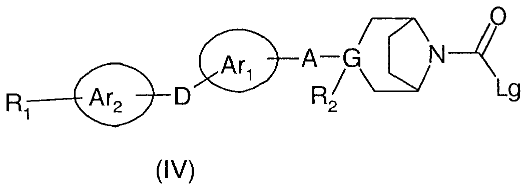 in which A, D, G, Ar1, Ar2, R1 and R2 are as defined in claim 1 and Lg is a leaving group,
is reacted with a compound of formula (V): 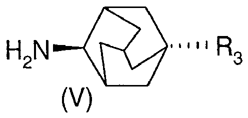 in which R3 is as defined in claim 1,
in the presence of a solvent and of a base.

8. A medicament, **characterized in that** it comprises a compound of formula (I) as claimed in any one of claims 1 to 6, or an addition salt of this compound with a pharmaceutically acceptable acid.

9. A pharmaceutical composition, **characterized in that** it comprises a compound of formula (I) as claimed in any one of claims 1 to 6, or a pharmaceutically acceptable salt, and also at least one pharmaceutically acceptable excipient.

10. A compound of formula (I) as claimed in any one of claims 1 to 6, for use in the treatment of obesity, diabetes, insulin resistance, metabolic syndrome, Cushing's syndrome, hypertension, atherosclerosis, cognition and dementia, glaucoma, osteoporosis and certain infectious diseases by increasing the effectiveness of the immune system.
